# EUROPEAN PATENT SPECIFICATION

(11) **EP 2 144 604 B1**
(45) Date of publication and mention of the grant of the patent: **21.09.2011**
(21) Application number: 08726169.9
(22) Date of filing: 27.02.2008
(51) Int. Cl.: A61K 31/16, A61K 31/351, A61K 31/381, A61K 31/403, A61K 31/405, A61K 31/4402, A61K 31/4439, A61P 1/16

(54) **Methods for the treatment of chronic viral hepatitis C using RO 113-0830**
Verfahren zur Behandlung von chronischer viraler Hepatitis C mithilfe von RO 113-0830
Procédés pour le traitement d'hépatite C virale chronique en utilisant RO 113-0830

(30) Priority: 28.02.2007 US 904322 P; 26.06.2007 US 937301 P
(43) Date of publication of application: 20.01.2010
(73) Proprietor: Conatus Pharmaceuticals, Inc., San Diego, CA 92121 (US)
(72) Inventor: SPADA, Alfred, P., Carisbad, CA 92009 (US)
(74) Representative: Jones Day
(86) International application number: PCT/US2008/002591
(87) International publication number: WO 2008/106166

(56) References cited:
- WO-A-99/61413
- WO-A-02/067870
- WO-A-2004/073599
- WO-A-2004/091603
- WO-A-2007/016390

## Description

Provided herein is a matrix metalloproteinase inhibitor for use in treating chronic viral hepatitis C.

Liver disease is an acute or chronic damage to the liver, usually caused by infection, injury, exposure to drugs or toxic compounds, alcohol, impurities in foods, and the abnormal build-up of normal substances in the blood, an autoimmune process, or by a genetic defect (such as haemochromatosis). Sometimes the exact cause of the injury may not be known. Liver disease can be classified as acute or chronic liver disease based in the duration of the disease. In acute liver disease, such as acute hepatitis and acute liver failure (ALF), the history of the disease does not exceed six months. Liver diseases of longer duration are classified as chronic liver disease.

The common liver diseases include cirrhosis, liver fibrosis, non-alcoholic fatty liver disease (NAFLD), non-alcoholic steatohepatitis (NASH), hepatic ischemia reperfusion injury, primary biliary cirrhosis (PBC), hepatitis, including viral and alcoholic hepatitis. Most common forms of viral hepatitis are hepatitis B and C (HBV and HCV, respectively). Chronic hepatitis may result in cirrhosis. Cirrhosis caused by chronic hepatitis C infection accounts for 8,000-12,000 deaths per year in the United States, and HCV infection is the leading indication for liver transplantation.

The death of liver cells through a process known as apoptosis is common in all forms of liver disease. Apoptosis of liver cells is linked to liver fibrosis and other liver disease. Prevention of excessive apoptosis liver cells is an important component in the treatment of acute and chronic liver disease (see, Guicciardi et al. Gut, 2005: 54, 1024-1033 and Ghavami et al., Med. Sci. Monit., 2005: 11(11): RA337-345).

The presence of active liver disease is often detected by the existence of elevated enzyme levels in the blood. Specifically, blood levels of ALT (alanine aminotransferase) and AST (aspartate aminotransferase), above clinically accepted normal ranges, are known to be indicative of on-going liver damage. Routine monitoring of liver disease patients for blood levels of ALT and AST is used clinically to measure progress of the liver disease while on medical treatment. Reduction of elevated ALT and AST to within the accepted normal range is taken as clinical evidence reflecting a reduction in the severity of the patients on-going liver damage. (Kim W. R. et al. Hepatology, 2008, accepted preprint available on line, accessed on publisher website 2/20/2008).

In light of the fact that liver diseases affect a large patient population worldwide, and has tragic effects on the affected patient, there remains a strong need to provide new effective pharmaceutical agents to treat liver disease.

Provided herein is a matrix metalloproteinase inhibitor for use in treating chronic viral hepatitis C. In certain embodiments, provided are uses for treatment of hepatitis C for patients who have failed therapy for hepatits C. In one embodiment, the uses provided herein reduce liver damage associated with chronic liver diseases. In one embodiment, the uses provided herein lower elevated levels of liver enzymes, such as elevated levels of ALT (alanine aminotransferase) and AST (aspartate aminotransferase).

In certain embodiments, the uses provided herein are for inhibiting hepatitis C virus (HCV) replication in a cell infected with hepatitis C virus. In certain embodiments, the uses provided herein are for inhibiting hepatitis C virus (HCV) replication in a patient infected with HCV.

The matrix metalloproteinase inhibitor for the uses provided herein is:

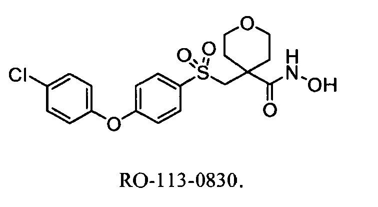

Also described are pharmaceutical compositions containing therapeutically effective amounts of one or more of compounds described herein and a pharmaceutically acceptable carrier, wherein the pharmaceutical compositions are useful in the prevention, treatment, or amelioration of one or more of the symptoms of liver diseases.

Further described is an article of manufacture containing packaging material, a compound or pharmaceutically acceptable derivative thereof described herein, which is used for treatment, prevention or amelioration of one or more symptoms associated with a liver disease, and a label that indicates that the compound or pharmaceutically acceptable derivative thereof is used for treatment, prevention or amelioration of one or more symptoms of a liver disease.

### Definitions

Unless defined otherwise, all technical and scientific terms used herein have the same meaning as is commonly understood by one of ordinary skill in the art. All patents, applications, published applications and other publications are incorporated by reference in their entirety. In the event that there are a plurality of definitions for a term herein, those in this section prevail unless stated otherwise.

As used herein "subject" is an animal, such as a mammal, including human, such as a patient.

As used herein, biological activity refers to the *in vivo* activities of a compound or physiological responses that result upon *in vivo* administration of a compound, composition or other mixture. Biological activity, thus, encompasses therapeutic effects and pharmacokinetic behaviour of such compounds, compositions and mixtures. Biological activities can be observed in *in vitro* systems designed to test for such activities.

As used herein, pharmaceutically acceptable derivatives of a compound include salts, esters, acetals, ketals, orthoesters, hemiacetals, hemiketals, acids, bases, solvates, hydrates or prodrugs thereof. Such derivatives may be readily prepared by those of skill in this art using known methods for such derivatization. The compounds produced may be administered to animals or humans without substantial toxic effects and either are pharmaceutically active or are prodrugs. Pharmaceutically acceptable salts include, but are not limited to, amine salts, such as but not limited to N,N'-dibenzylethylenediamine, chloroprocaine, choline, ammonia, diethanolamine and other hydroxyalkylamines, ethylenediamine, N-methylglucamine, procaine, N-benzylphenethylamine, 1-para-chlorobenzyl-2-pyrrolidin-1'-ylmethylbenzimidazole, diethylamine and other alkylamines, piperazine and tris(hydroxymethyl)aminomethane; alkali metal salts, such as but not limited to lithium, potassium and sodium; alkali earth metal salts, such as but not limited to barium, calcium and magnesium; transition metal salts, such as but not limited to zinc; and inorganic salts, such as but not limited to, sodium hydrogen phosphate and disodium phosphate; and also including, but not limited to, salts of mineral acids, such as but not limited to hydrochlorides and sulfates; and salts of organic acids, such as but not limited to acetates, lactates, malates, tartrates, citrates, ascorbates, succinates, butyrates, valerates, mesylates, and fumarates. Pharmaceutically acceptable esters include, but are not limited to, alkyl, alkenyl, alkynyl, aryl, aralkyl, and cycloalkyl esters of acidic groups, including, but not limited to, carboxylic acids, phosphoric acids, phosphinic acids, sulfonic acids, sulfinic acids and boronic acids. Pharmaceutically acceptable solvates and hydrates are complexes of a compound with one or more solvent or water molecules, or 1 to about 100, or 1 to about 10, or one to about 2, 3 or 4, solvent or water molecules.

As used herein, treatment means any manner in which one or more of the symptoms of a disease or disorder are ameliorated or otherwise beneficially altered. Treatment also encompasses any pharmaceutical use of the compositions herein, such as use for treating a liver disease.

As used herein, amelioration of the symptoms of a particular disorder by administration of a particular compound or pharmaceutical composition refers to any lessening, whether permanent or temporary, lasting or transient that can be attributed to or associated with administration of the composition.

As used herein, and unless otherwise indicated, the terms "manage," "managing" and "management" encompass preventing the recurrence of the specified disease or disorder in a patient who has already suffered from the disease or disorder, and/or lengthening the time that a patient who has suffered from the disease or disorder remains in remission. The terms encompass modulating the threshold, development and/or duration of the disease or disorder, or changing the way that a patient responds to the disease or disorder.

It is to be understood that the compounds described herein may contain chiral centers. Such chiral centers may be of either the (R) or (S) configuration, or may be a mixture thereof. Thus, the compounds described herein may be enantiomerically pure, or be stereoisomeric or diastereomeric mixtures. As such, one of skill in the art will recognize that administration of a compound in its (R) form is equivalent, for compounds that undergo epimerization *in vivo,* to administration of the compound in its (S) form.

As used herein, substantially pure means sufficiently homogeneous to appear free of readily detectable impurities as determined by standard methods of analysis, such as thin layer chromatography (TLC), gel electrophoresis, high performance liquid chromatography (HPLC) and mass spectrometry (MS), used by those of skill in the art to assess such purity, or sufficiently pure such that further purification would not detectably alter the physical and chemical properties, such as enzymatic and biological activities, of the substance. Methods for purification of the compounds to produce substantially chemically pure compounds are known to those of skill in the art. A substantially chemically pure compound may, however, be a mixture of stereoisomers. In such instances, further purification might increase the specific activity of the compound. The instant disclosure is meant to include all such possible isomers, as well as, their racemic and optically pure forms. Optically active (+) and (-), (R)- and (S)-, or (D)- and (L)-isomers may be prepared using chiral synthons or chiral reagents, or resolved using conventional techniques, such as reverse phase HPLC. When the compounds described herein contain olefinic double bonds or other centers of geometric asymmetry, and unless specified otherwise, it is intended that the compounds include both E and Z geometric isomers. Likewise, all tautomeric forms are also intended to be included.

In certain embodiments, the compound described herein is "stereochemically pure." A stereochemically pure compound or has a level of stereochemical purity that would be recognized as "pure" by those of skill in the art. In certain embodiments, "stereochemically pure" designates a compound that is substantially free of alternate isomers. In particular embodiments, the compound is 85%, 90%, 91%, 92%, 93%, 94%, 95%, 96%, 97%, 98%, 99%, 99.5% or 99.9% free of other isomers.

As used herein, "therapy for liver disease" refers to a treatment with any medication known and available in the market or being developed for the treatment of liver disease. For example, therapy of hepatitis C refers to treatment of the patient with drugs available in the market for HCV treatment. Several exemplary drugs are described in the section on "Combination Therapy" *infra.*

As used herein, "patients who have failed therapy" refers to the patient population described in Section 4.3, *infra* and includes patients that has been previously treated for a liver disease with any of the drugs currently available in the market and either did not respond to the therapy or had temporary relief from the liver disease.

As used herein "liver damage" refers to an acute or chronic damage to the liver, usually caused by infection, injury, exposure to drugs or toxic compounds, alcohol, impurities in foods, and the abnormal build-up of normal substances in the blood, an autoimmune process, graft rejection related with transplantation or by a genetic defect (such as haemochromatosis). The damage to liver includes, but is not limited to inflammation, scarring of liver tissue and fibrosis.

As used herein, the term "in combination" refers to the use of more than one therapies (e.g., an MMP and interferon). The use of the term "in combination" does not restrict the order in which therapies (e.g., an MMP and interferon) are administered to a subject with a disorder. A first therapy (e.g., an MMP inhibitor) can be administered prior to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy (e.g., interferon) to a subject with a disorder.

As used herein, the term "synergistic" refers to a combination of an MMP inhibitor and a second agent, such as interferon,, which is more effective than the additive effects of the administration of the two compounds as monotherapies. A synergistic effect of a combination of therapies (e.g., a combination of an MMP and interferon) permits the use of lower dosages of one or more of the therapies and/or less frequent administration of the therapies to a subject with a disorder. The ability to utilize lower dosages of a therapy (e.g., an MMP and interferon) and/or to administer the therapy less frequently reduces the toxicity associated with the administration of the therapy to a subject without reducing the efficacy of the therapy in the prevention or treatment of a disorder. In addition, a synergistic effect can result in improved efficacy of agents in the prevention or treatment of a disorder. Finally, a synergistic effect of a combination of therapies (e.g., a combination of an MMP and interferon) may avoid or reduce adverse or unwanted side effects associated with the use of either therapy alone.

As used herein, the terms "other agent" or "second agent" refer to any agent or combination of agents that can be used for treatment of liver disease in combination with the MMP inhibitors described herein. In certain embodiments, the other agent or second agent is anti-hepatitis C virus interferon, an anti-hepatitis C virus polymerase inhibitor, an anti-hepatitis C virus protease inhibitor or a combination thereof.

As used herein, the terms "elevated levels of liver enzymes" or "raised levels of liver enzymes" refer to the level of liver enzymes in blood that are in excess of the normal clinically accepted range for liver enzymes in blood. The compounds provided herein reduce the elevated liver enzyme levels to the normal clinically accepted levels of liver enzymes in blood. Methods for measuring the level of liver enzymes are well known in the art (see, e.g., Jeong S. Y. et al. Sandwich ELISA for measurement of cytosolic aspartate aminotransferase in sera from patients with liver diseases, Clin Chem., 2003; 49(5):826 9 and Burin des Roziers N. et al. A microtiter plate assay for measurement of serum alanine aminotransferase in blood donors, Transfusion., 1995; 35(4):331 4 ).

### Compounds for use

The compounds for use provided herein are matrix metalloproteinase inhibitors (MMP inhibitors). Several MMP inhibitors have been reported in the literature. Certain exemplary MMP inhibitors for use in the methods herein are described by Fisher et al. in Cancer Metastasis Rev., (2006) 25: 115-136; Rao in Current Pharmaceutical Design, 2005, 11, 295-322 295, Bender et al. in U.S. patent no. 5,932,595; Watanabe in U.S. patent nos. 6,207,698 and 6,831,178; Levin et al. in U.S. patent no. 6,225,311; Purder et al. in WO 2007/016390 and Alwayn et al. Am J Physiol Gastrointest Liver Physiol 291: G1011-G1019, 2006.

Described herein are compounds selected from 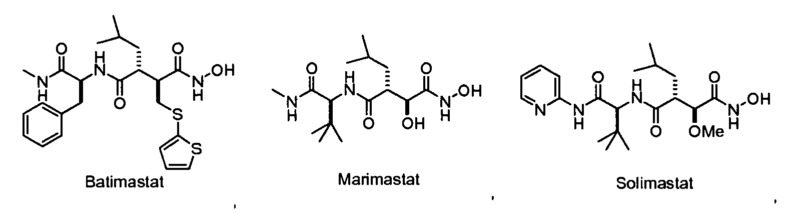 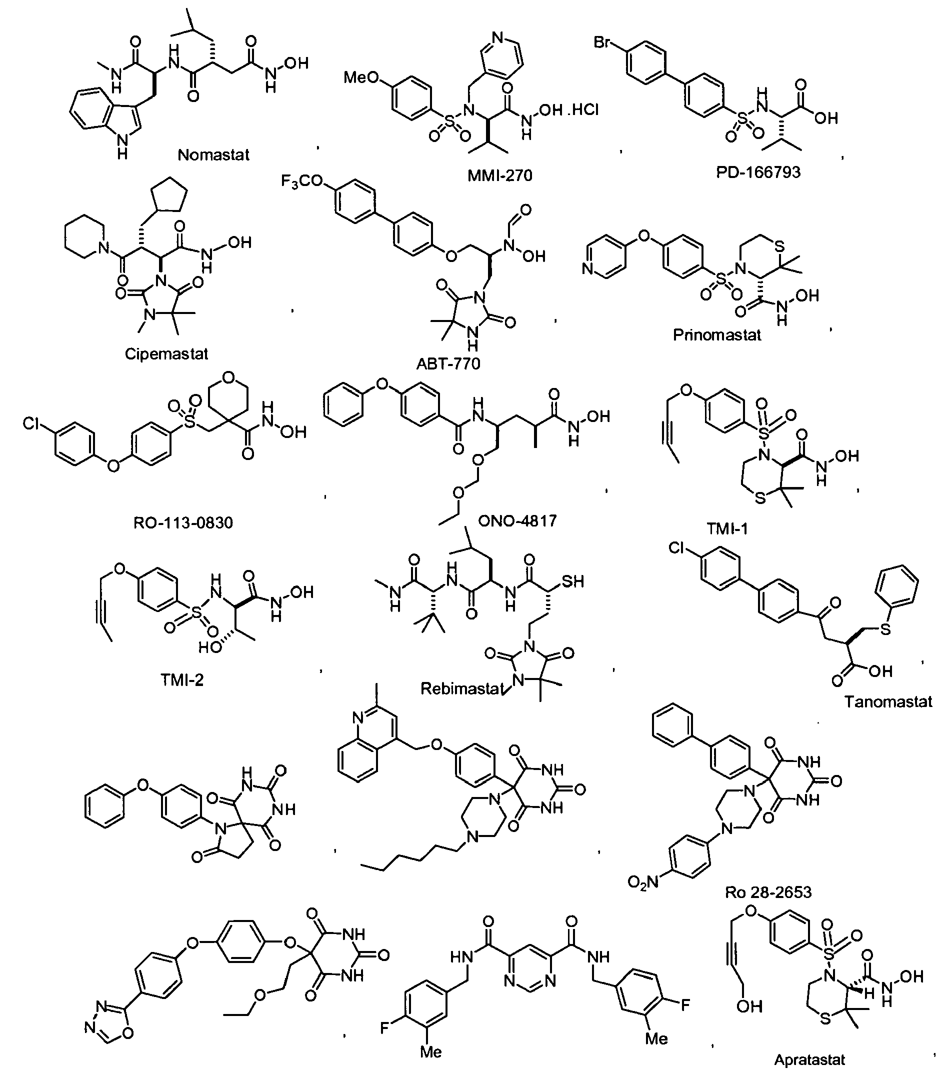 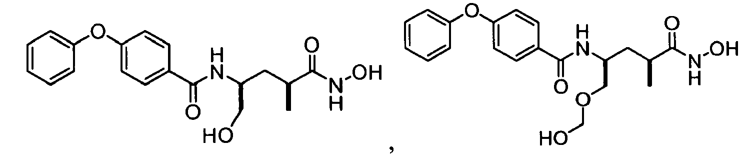 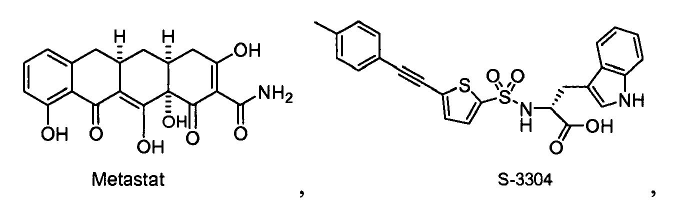 XL784 and a pharmaceutically acceptable derivative thereof.

Also described herein are compounds selected from 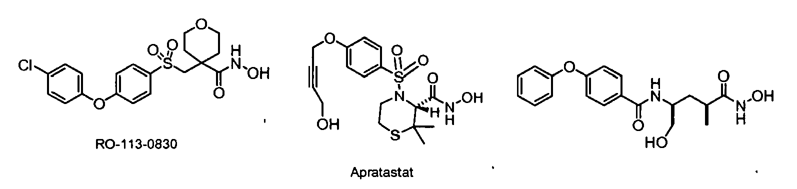 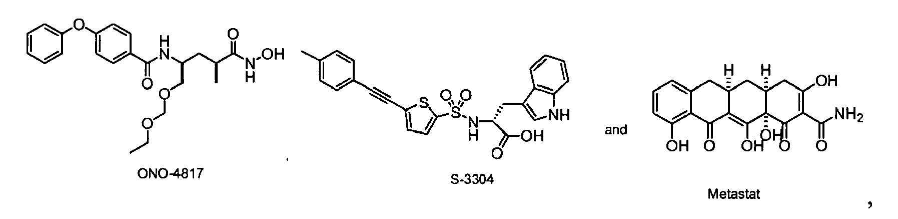 or a pharmaceutically acceptable derivative thereof.

The compound for use provided herein is

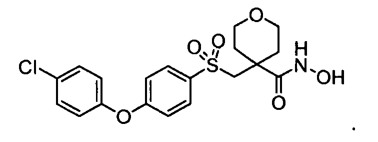

Also described herein is the compound 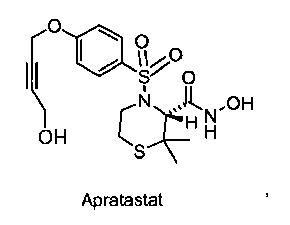 or a pharmaceutically acceptable derivative thereof.

Also described herein is the compound 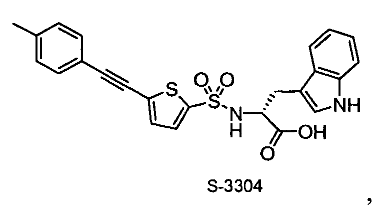 or a pharmaceutically acceptable derivative thereof.

In certain embodiments, the compounds described herein have efficacy in models of acute liver disease following oral administration of from 0.001 - 1000 mg/Kg. In certain embodiments, the compounds described herein have efficacy in models of acute liver disease following oral administration of from 0.01 - 100 mg/Kg.

### Uses for treatment

The uses provided herein are for treatment of a chronic liver disease. In one embodiment, the uses are for reducing liver damage associated with chronic liver disease. Without being bound to any particular theory, it is believed that the MMP inhibitors in the uses provided herein can act in part by inhibiting the signalling cascade of TNF-α. Thus, in one embodiment, provided herein are uses for inhibiting the signalling cascade of TNF-α by administering a compound described herein. Further described herein is the treatment of acute and/or chronic liver disease. Further described herein is the treatment of an acute liver disease.

Also described herein is a liver disease which is a disorder that results from an injury to the liver. For example, injury to the liver is caused by toxins, including alcohol, some drugs, impurities in foods, and the abnormal build-up of normal substances in the blood; or by an infection or by an autoimmune disorder. Sometimes the exact cause of the injury is not known.

The uses provided herein are for treating a liver disease which is chronic viral hepatitis C. Also described herein is a liver disease which includes, but is not limited to cirrhosis, liver fibrosis, non-alcoholic fatty liver disease (NAFLD), non-alcoholic steatohepatitis (NASH), hepatic ischemia reperfusion injury, hepatitis, including viral and alcoholic hepatitis and primary biliary cirrhosis. In one embodiment, the liver disease is manifested by raised liver enzymes (e.g., ALT and AST), pathological evidence of on going liver damage as a result of steatosis (fatty liver), fibrosis, and/or cirrhosis. In one embodiment, NASH is manifested by raised liver enzymes (e.g., ALT and AST), pathological evidence of steatosis (fatty liver), fibrosis, and/or cirrhosis.

Also described herein is treatment of fatty liver (also called hepatic steatosis), including non-alcoholic fatty liver disease. Fatty liver is defined as an excessive accumulation of triglyceride inside the liver cells. For example, in patients with non-alcoholic fatty liver disease, liver contains more that about 5% of the total weight of the liver or more than 30% of liver cells in a liver lobule are with fat deposit. The most common causes of non-alcoholic fatty liver are obesity, diabetes, and elevated serum triglyceride levels. Other causes include malnutrition, hereditary disorders of metabolism (such as the glycogen storage diseases, and drugs (such as corticosteroids, tetracycline and aspirin). In certain cases, fatty liver produces no symptoms. In other cases, fatty liver results in jaundice (a yellowish discoloration of the skin and the whites of the eyes), nausea, vomiting, pain, and abdominal tenderness. The uses described herein are useful in treating one or more of the symptoms of non-alcoholic fatty liver disease.

Fatty liver with liver inflammation not caused by alcohol is known as non-alcoholic steatohepatitis or NASH. NASH can be caused by any of the causes mentioned above as possible causes of non-alcoholic fatty liver disease. Described herein are uses for treatment of NASH.

The uses provided herein are for treating chronic viral hepatitis C. Also described are uses for treatment of hepatitis or inflammation of the liver, including viral and alcoholic hepatitis. The viral hepatitis can be acute or chronic. In certain embodiments, the acute viral hepatitis is caused by hepatitis A, B, C, D or E virus. In other embodiments, the acute viral hepatitis is caused by hepatitis B or C virus. The uses provided are for treatment of chronic viral hepatitis C. Described is chronic viral hepatitis caused by hepatitis B or C virus. In certain embodiments, the uses provided are for treatment of hepatitis C patients who have failed therapy for hepatitis C. Exemplary methods of treatment of hepatits C are described by Strader et al., in Hepatology, 39 (4), 2004.

In certain embodiments, the patient has never received therapy or prophylaxis for HCV infection. In further embodiments, the patient has previously received therapy or prophylaxis for HCV infection. For instance, in certain embodiments, the patient has not responded to HCV therapy. As known in the art, under current interferon therapy, up to 50% or more HCV patients do not respond to therapy. In certain embodiments, the patient can be a patient that received therapy but continued to suffer from HCV or one or more symptoms thereof. In certain embodiments, the patient can be a patient that received therapy but failed to achieve a sustained response. In certain embodiments, the patient has received therapy for HCV infection but has failed show a 2 log₁₀ decline in HCV RNA levels after 12 weeks of therapy. It is believed that patients who have not shown more than 2 log₁₀ reduction in serum HCV RNA after 12 weeks of therapy have a 97-100% chance of not responding.

In certain embodiments, the patient is a patient that discontinued HCV therapy because of one or more adverse events associated with the therapy. In certain embodiments, the patient is a patient where current therapy is not indicated. For instance, certain therapies for HCV are associated with neuropsychiatric events. Interferon (IFN)-α plus ribavirin is associated with a high rate of depression. Depressive symptoms have been linked to a worse outcome in a number of medical disorders. Life-threatening or fatal neuropsychiatric events, including suicide, suicidal and homicidal ideation, depression, relapse of drug addiction/overdose, and aggressive behavior have occurred in patients with and without a previous psychiatric disorder during HCV therapy. Interferon-induced depression is a limitation for the treatment of chronic hepatitis C, especially for patients with psychiatric disorders. Psychiatric side effects are common with interferon therapy and responsible for about 10% to 20% of discontinuations of current therapy for HCV infection.

Accordingly, provided are uses for treating or preventing hepatits C in patients where the risk of neuropsychiatric events, such as depression, contraindicates treatment with current HCV therapy. Also provided are uses for treating or preventing hepatitis C in patients where a neuropsychiatric event, such as depression, or risk of such indicates discontinuation of treatment with current HCV therapy. Further provided are uses for treating or preventing hepatitis C in patients where a neuropsychiatric event, such as depression, or risk of such indicates dose reduction of current HCV therapy.

Current therapy is also contraindicated in patients that are hypersensitive to interferon or ribavirin, or both, or any other component of a pharmaceutical product for administration of interferon or ribavirin. Current therapy is not indicated in patients with hemoglobinopathies (e.g., thalassemia major, sickle-cell anemia) and other patients at risk from the hematologic side effects of current therapy. Common hematologic side effects are include bone marrow suppression, neutropenia and thrombocytopenia. Furthermore, ribavirin is toxic to red blood cells and is associated with hemolysis. Accordingly, the uses provided herein are useful in patients hypersensitive to interferon or ribavirin, or both, patients with a hemoglobinopathy, for instance, thalassemia major patients and sickle-cell anemia patients, and other patients at risk from the hematologic side effects of current therapy.

In certain embodiments the patient has received HCV therapy and discontinued that therapy prior to administration of a compound provided herein. In further embodiments, the patient has received therapy and continues to receive that therapy along with administration of a compound provided herein. The compounds herein can be co-administered with other therapy for HCV according to the judgment of one of skill in the art. In certain embodiments, the compounds herein can be co-administered with a reduced dose of the other therapy for HCV.

In certain embodiments, provided are uses for treating a patient that is refractory to treatment with interferon. For instance, in some embodiments, the patient can be a patient that has failed to respond to treatment with one or more agents selected from the group consisting of interferon, interferon α, pegylated interferon α, interferon plus ribavirin, interferon α plus ribavirin and pegylated interferon α plus ribavirin. In some embodiments, the patient can be a patient that has responded poorly to treatment with one or more agents selected from the group consisting of interferon, interferon α, pegylated interferon α, interferon plus ribavirin, interferon α plus ribavirin and pegylated interferon α plus ribavirin.

In one embodiment, chronic HCV infection is manifested by raised liver enzymes (e.g., ALT, AST), persistent (e.g., greater than six months) HCV RNA levels, and/or histological evidence of liver damage, fibrosis, and/or cirrhosis. In one embodiment, the uses provided herein lower elevated liver enzyme levels, such as ALT and AST levels. Methods for measuring the level of liver enzymes are well known in the art (see, e.g., Jeong S. Y. et al. Sandwich ELISA for measurement of cytosolic aspartate aminotransferase in sera from patients with liver diseases, Clin Chem., 2003; 49(5):826 9 and Burin des Roziers N. et al. A microtiter plate assay for measurement of serum alanine aminotransferase in blood donors, Transfusion., 1995; 35(4):331 4,). In one embodiment, the elevated level of one or more liver enzyme, such as ALT or AST, or the total amount of elevated level of liver enzyme above the normal range is reduced by more than about 90% or more than 95%. In one embodiment, the elevated level of one or more liver enzyme, such as elevated levels of ALT or AST, or the total amount of elevated liver enzyme is reduced by at least 95%, at least 90%, at least 80%, at least 70%, at least 60%, at least 50%, at least 40%, at least 30%, at least 20%, at least 10%, at least 5%, at least 2% or at least 1%.

In certain embodiment, provided herein are uses for treating patients infected with hepatitis C virus and have normal serum aminotransferase levels. It has been reported that up to 60% of HCV-infected first-time blood donors and injection drug users have normal levels of ALT (*see,* Strader et al., in Hepatology, 39 (4), 2004). In one embodiment, a person is considered to have normal ALT levels when there have been two or more determinations identified to be in the normal range of a licensed laboratory over six or more months. It is known in the art that biopsies of those with normal aminotransferase values have revealed bridging fibrosis or cirrhosis in 1% to 10% of cases, and at least portal fibrosis in a greater proportion (Strader et al., in Hepatology, 39 (4), 2004). In one embodiment, the compounds provided herein are useful in treating such patients.

In certain embodiments, the uses provided herein are for inhibiting hepatitis C virus (HCV) replication in a cell infected with hepatitis C virus. In certain embodiments, a therapeutically effective amount of the compound is an amount sufficient to cause a detectable decrease in HCV replication. The compound for use is RO-113-0830. Methods for detection of HCV replication are known to one of skill in the art and include the HCV replicon assay. An exemplary assay is described by Pietschmann, T. et al., J. Virol. 76, 2002, 4008-4021. In certain embodiments, HCV replication is inhibited at least about 20%, at least about 30%, at least about 40%, at least about 50%, at least about 75%, at least about 90% or more.

In another aspect, the uses provided herein are for inhibiting HCV replication in a patient infected with hepatitis C virus. The uses involve the step of administering to the patient an effective amount of a compound provided herein. The uses involve the step of administering to the patient an effective amount of RO-113-0830.

Also described are uses for treatment of alcoholic hepatitis. Alcoholic hepatitis (steatohepatitis) is a combination of fatty liver, diffuse liver inflammation, and liver necrosis, in certain embodiments, focal necrosis, all in various degrees of severity.

In one embodiment, the uses provided herein are for treating liver fibrosis, lobular hepatitis and/or periportal bridging necrosis in a patient. Liver fibrosis is the excessive accumulation of extracellular matrix proteins including collagen that occurs in most types of chronic liver diseases. In certain embodiments, advanced liver fibrosis results in cirrhosis and liver failure. In one embodiment, the uses provided herein are for reducing the level of fibrosis, lobular hepatitis and/or periportal bridging necrosis in a patient. Methods for measuring liver histologies such as changes in the extent of fibrosis, lobular hepatitis, and periportal bridging necrosis are well known in the art. For example, several non-invasive tests for liver fibrosis are described in Hepatology, 2006, 43(2):S113-S120. Hepatology, 2007, 45(1):242-249 describes the measurement and treatment of liver fibrosis. Wright M. et al. describe measurement and determinants of the natural history of liver fibrosis in hepatitis C virus infection: a cross sectional and longitudinal study in Gut. 2003; 52(4):574 9.
In the uses provided herein, liver fibrosis is caused by hepatitis. Also described is liver fibrosis caused by chemical exposure, bile duct obstruction, autoimmune disease, obstruction of outflow of blood from the liver, heart and blood vessel disturbance, α1-antitrypsin deficiency, high blood galactose level, high blood tyrosine level, glycogen storage disease, diabetes, malnutrition, Wilson Disease or hemochromatosis.

In one embodiment, the level of fibrosis, which is the formation of fibrous tissue, fibroid or fibrous degeneration, is reduced by more that about 90%. In one embodiment, the level of fibrosis is reduced by at least 90%, at least 80%, at least 70%, at least 60%, at least 50%, at least 40%, at least 30%, at least 20%, at least 10%, at least 5% or at least 2%.

In one embodiment, the compounds provided herein reduce the level of fibrogenesis. Liver fibrogenesis is the process leading to the deposition of an excess of extracellular matrix components in the liver known as fibrosis. It is observed in a number of conditions such as chronic viral hepatitis B and C, alcoholic liver disease, drug-induced liver disease, hemochromatosis, auto-immune hepatitis, Wilson disease, primary biliary cirrhosis, sclerosing cholangitis, liver schistosomiasis and others. In one embodiment, the level of fibrogenesis is reduced by more that about 90%. In one embodiment, the level of fibrogenesis is reduced by at least 90%, at least 80%, at least 70%, at least 60%, at least 50%, at least 40%, at least 30%, at least 20%, at least 10%, at least 5% or at least 2%.

In one embodiment, the level of lobular hepatitis, wherein foci of inflammatory cells are also present in the sinusoids of the lobule is reduced by more that about 99% or 95%. In another embodiment, the level of lobular hepatitis is reduced by at least 90%, at least 80%, at least 70%, at least 60%, at least 50%, at least 40%, at least 30%, at least 20%, at least 10%, at least 5%, at least 2% or at least 1%. In yet another embodiment, the level of periportal bridging necrosis is reduced by more than about 90%. In yet another embodiment, the level of periportal bridging necrosis is reduced by at least 90%, at least 80%, at least 70%, at least 60%, at least 50%, at least 40%, at least 30%, at least 20%, at least 10%, at least 5%, at least 2% or at least 1%.

In one embodiment, the uses provided herein are for treating of cirrhosis. In certain embodiments, symptoms of cirrhosis include, but are not limited to, portal hypertension, abnormal nerve function, ascites (build-up of fluid in the abdominal cavity), breast enlargement in men, coughing up or vomiting blood, curling of fingers (Dupuytren contracture of the palms), gallstones, hair loss, itching, jaundice, kidney failure, liver encephalopathy, muscle loss, poor appetite, redness of palms, salivary gland enlargement in cheeks, shrinking of testes, small spider-like veins in skin, weakness, weight loss, spider angiomas (a central arteriole from which numerous small branching vessels radiate), encephalopathy, and asterixis (flapping tremor). Symptoms of cirrhosis vary, depending on severity and individuals. In certain embodiments, mild cirrhosis may not exhibit any symptoms at all.

In the uses provided herein, the cause of cirrhosis is hepatitis C. Other described causes of cirrhosis include, use of certain drugs, chemical exposure, bile duct obstruction, autoimmune diseases, obstruction of outflow of blood from the liver (i.e., Budd-Chiari syndrome), heart and blood vessel disturbances, alpha1-antitrypsin deficiency, high blood galactose levels, high blood tyrosine levels, glycogen storage disease, diabetes, malnutrition, hereditary accumulation of too much copper (Wilson Disease) or iron (hemochromatosis). A further described cause of cirrhosis is alcohol abuse.

In one embodiment, the uses provided herein are for reducing the level of cirrhosis. In one embodiment, cirrhosis is characterized pathologically by loss of the normal microscopic lobular architecture, with fibrosis and nodular regeneration. Methods for measuring the extent of cirrhosis are well known in the art. In one embodiment, the level of cirrhosis is reduced by about 5%-100%. In one embodiment, the level of cirrhosis is reduced by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or 100% in the patient.

In certain embodiments, the uses for treatment of cirrhosis involve administration of an MMP inhibitor provided herein.

Also described are uses for treatment of primary biliary cirrhosis (PBC). Primary biliary cirrhosis begins with inflammation of the bile ducts inside the liver. The inflammation blocks the flow of bile out of the liver; thus, bile remains in the liver cells or spills over into the bloodstream. As inflammation spreads from the bile ducts to the rest of the liver, a latticework of scar tissue develops throughout the liver. Described are uses for treatment of PBC in women aged 35 to 60. The PBC can be caused by an autoimmune disorder. Primary biliary cirrhosis can occur in association with rheumatoid arthritis, scleroderma, or autoimmune thyroiditis. The uses described herein are useful in treating one or more of the symptoms of primary biliary cirrhosis.

Also described are uses for treatment of hepatic ischemia reperfusion injury. Ischemia can occur in the liver due to several pathological conditions, such as liver transplantation, cardiogenic or hemodynamic shock, and liver resection for trauma or tumor. When the blood circulation is reestablished (reperfusion), the rapid increase in oxygen concentration leads to the production of reactive oxygen species, which in turn cause a generalized damage of hepatic cells (both necrosis and apoptosis) resulting in ischemia-reperfusion (IR) injury in the liver. The described uses for treatment of hepatic ischemia reperfusion injury can involve administration of an MMP inhibitor described herein, with a proviso that the MMP inhibitor is other than ONO-4817. For example, the described uses for treatment of hepatic ischemia reperfusion injury can involve administration ofRO-113-0830.

As known to one of skill in the art, excess apoptosis of liver cells is linked to liver fibrosis and other liver disease. Thus, prevention or suppression of excessive apoptosis liver cells is an important component in the treatment of acute and chronic liver disease. Apoptosis occurs mainly via a two signalling pathways: a death receptor mediated extrinsic pathway or a mitochondria mediated intrinsic pathway. The extrinsic pathway originates at the plasma membrane following the engagement of a family of cytokine receptors named death receptors (such as tumour necrosis factor receptor 1 (TNF-R1), Fas/CD95, and tumour necrosis factor related apoptosis inducing ligand receptors 1 and 2 (TRAIL-R1 and TRAIL-R2)) by their cognate ligands (TNF-, Fas ligand (FasL)/CD95L, TRAIL). *See*, Guicciardi et al. Gut, 2005: 54, 1024-1033 and Ghavami et al., Med. Sci. Monit., 2005: 11(11): RA337-345. In certain embodiments, the MMP inhibitors provided herein block damage of liver cells by preventing or suppressing apoptosis. In certain embodiments, the compounds provided herein inhibit a signalling cascade of α-Fas. In certain embodiments, the compounds provided herein inhibit a signalling cascade initiated by TNF-α. Without being bound to any particular theory, it is believed that in certain embodiments, the prevention or suppression of excessive apoptosis of liver cells by compounds provided herein contributes to reducing liver damage associated with acute and/or chronic liver disease.

### Preparation of the compounds

The compounds for use described herein can be prepared by using routine synthetic procedures, including procedures described in Bender et al. in U.S. patent no. 5,932,595 and Watanabe in U.S. patent nos. 6,207,698 and 6,831,178. An exemplary method for preparation of RO-113-0830 is described in Example 1.

### Formulation of pharmaceutical compositions

The pharmaceutical compositions described herein contain therapeutically effective amounts of one or more of compounds described herein that are useful in the prevention, treatment, or amelioration of one or more of the symptoms of liver diseases and a pharmaceutically acceptable carrier.

The compounds are formulated into suitable pharmaceutical preparations such as solutions, suspensions, tablets, dispersible tablets, pills, capsules, powders, sustained release formulations or elixirs, for oral administration or in sterile solutions or suspensions for parenteral administration, as well as transdermal patch preparation and dry powder inhalers. For example, the compounds described above are formulated into pharmaceutical compositions using techniques and procedures well known in the art (*see*, *e.g*., Remington's Pharmaceutical Sciences, 20th eds., Mack Publishing, Easton PA (2000)).

In the compositions, effective concentrations of one or more compounds or pharmaceutically acceptable derivatives is (are) mixed with a suitable pharmaceutical carrier or vehicle. The compounds may be derivatized as the corresponding salts, esters, acids, bases, solvates, hydrates or prodrugs prior to formulation, as described above. The concentrations of the compounds in the compositions are effective for delivery of an amount, upon administration, that treats, prevents, or ameliorates one or more of the symptoms of liver diseases.

The compositions can be formulated for single dosage administration. To formulate a composition, the weight fraction of compound is dissolved, suspended, dispersed or otherwise mixed in a selected vehicle at an effective concentration such that the treated condition is relieved or ameliorated. Pharmaceutical carriers or vehicles suitable for administration of the compounds provided herein include any such carriers known to those skilled in the art to be suitable for the particular mode of administration.

In addition, the compounds may be formulated as the sole pharmaceutically active ingredient in the composition or may be combined with other active ingredients. Liposomal suspensions, including tissue-targeted liposomes, such as tumor-targeted liposomes, may also be suitable as pharmaceutically acceptable carriers. These may be prepared according to methods known to those skilled in the art. For example, liposome formulations may be prepared as known in the art. Briefly, liposomes such as multilamellar vesicles (MLV's) may be formed by drying down egg phosphatidyl choline and brain phosphatidyl serine (7:3 molar ratio) on the inside of a flask. A solution of a compound provided herein in phosphate buffered saline (PBS) lacking divalent cations is added and the flask shaken until the lipid film is dispersed. The resulting vesicles are washed to remove unencapsulated compound, pelleted by centrifugation, and then resuspended in PBS.

The active compound is included in the pharmaceutically acceptable carrier in an amount sufficient to exert a therapeutically useful effect in the absence of undesirable side effects on the patient treated. The therapeutically effective concentration may be determined empirically by testing the compounds in *in vitro* and *in vivo* systems known in the art and then extrapolated therefrom for dosages for humans.

The concentration of active compound in the pharmaceutical composition will depend on absorption, inactivation and excretion rates of the active compound, the physicochemical characteristics of the compound, the dosage schedule, and amount administered as well as other factors known to those of skill in the art. For example, the amount that is delivered is sufficient to ameliorate one or more of the symptoms of liver diseases.

For example, a therapeutically effective dosage should produce a serum concentration of active ingredient of from about 0.1 ng/ml to about 50-100 µg/ml. The pharmaceutical compositions, in certain embodiments, should provide a dosage of from about 0.001 mg to about 2000 mg of compound per kilogram of body weight per day. Pharmaceutical dosage unit forms are prepared to provide from about 1 mg to about 1000 mg and from about 10 to about 500 mg of the essential active ingredient or a combination of essential ingredients per dosage unit form.

The active ingredient may be administered at once, or may be divided into a number of smaller doses to be administered at intervals of time. It is understood that the precise dosage and duration of treatment is a function of the disease being treated and may be determined empirically using known testing protocols or by extrapolation from *in vivo* or *in vitro* test data. It is to be noted that concentrations and dosage values may also vary with the severity of the condition to be alleviated. It is to be further understood that for any particular patient, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions.

Pharmaceutically acceptable derivatives include acids, bases, salts, esters, hydrates, solvates and prodrug forms. The derivative is selected such that its pharmacokinetic properties are superior to the corresponding neutral compound.

Thus, effective concentrations or amounts of one or more of the compounds described herein or pharmaceutically acceptable derivatives thereof are mixed with a suitable pharmaceutical carrier or vehicle for systemic, topical or local administration to form pharmaceutical compositions. Compounds are included in an amount effective for ameliorating one or more symptoms of, or for treating or preventing liver diseases. The concentration of active compound in the composition will depend on absorption, inactivation, excretion rates of the active compound, the dosage schedule, amount administered, particular formulation as well as other factors known to those of skill in the art.

The compositions are intended to be administered by a suitable route, including orally, parenterally, rectally, topically and locally. For oral administration, capsules and tablets can be used. The compositions are in liquid, semi-liquid or solid form and are formulated in a manner suitable for each route of administration. In one embodiment, modes of administration include parenteral and oral modes of administration. In certain embodiments, oral administration is contemplated.

Solutions or suspensions used for parenteral, intradermal, subcutaneous, or topical application can include any of the following components: a sterile diluent, such as water for injection, saline solution, fixed oil, polyethylene glycol, glycerine, propylene glycol, dimethyl acetamide or other synthetic solvent; antimicrobial agents, such as benzyl alcohol and methyl parabens; antioxidants, such as ascorbic acid and sodium bisulfite; chelating agents, such as ethylenediaminetetraacetic acid (EDTA); buffers, such as acetates, citrates and phosphates; and agents for the adjustment of tonicity such as sodium chloride or dextrose. Parenteral preparations can be enclosed in ampoules, disposable syringes or single or multiple dose vials made of glass, plastic or other suitable material.

In instances in which the compounds exhibit insufficient solubility, methods for solubilizing compounds may be used. Such methods are known to those of skill in this art, and include, but are not limited to, using cosolvents, such as dimethylsulfoxide (DMSO), using surfactants, such as TWEEN®, or dissolution in aqueous sodium bicarbonate.

Upon mixing or addition of the compound(s), the resulting mixture may be a solution, suspension, emulsion or the like. The form of the resulting mixture depends upon a number of factors, including the intended mode of administration and the solubility of the compound in the selected carrier or vehicle. The effective concentration is sufficient for ameliorating the symptoms of the disease, disorder or condition treated and may be empirically determined.

The pharmaceutical compositions can be for administration to humans and animals in unit dosage forms, such as tablets, capsules, pills, powders, granules, sterile parenteral solutions or suspensions, and oral solutions or suspensions, and oil-water emulsions containing suitable quantities of the compounds or pharmaceutically acceptable derivatives thereof. The pharmaceutically therapeutically active compounds and derivatives thereof are formulated and administered in unit-dosage forms or multiple-dosage forms. Unit-dose forms as used herein refer to physically discrete units suitable for human and animal patients and packaged individually as is known in the art. Each unit-dose contains a predetermined quantity of the therapeutically active compound sufficient to produce the desired therapeutic effect, in association with the required pharmaceutical carrier, vehicle or diluent. Examples of unit-dose forms include ampoules and syringes and individually packaged tablets or capsules. Unit-dose forms may be administered in fractions or multiples thereof. A multiple-dose form is a plurality of identical unit-dosage forms packaged in a single container to be administered in segregated unit-dose form. Examples of multiple-dose forms include vials, bottles of tablets or capsules or bottles of pints or gallons. Hence, multiple dose form is a multiple of unit-doses which are not segregated in packaging.

Sustained-release preparations can also be prepared. Suitable examples of sustained-release preparations include semipermeable matrices of solid hydrophobic polymers containing the compound provided herein, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides, copolymers of L-glutamic acid and ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated compound remain in the body for a long time, they may denature or aggregate as a result of exposure to moisture at 37 °C, resulting in a loss of biological activity and possible changes in their structure. Rational strategies can be devised for stabilization depending on the mechanism of action involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions

Dosage forms or compositions containing active ingredient in the range of 0.005% to 100% with the balance made up from non-toxic carrier may be prepared. For oral administration, a pharmaceutically acceptable non-toxic composition is formed by the incorporation of any of the normally employed excipients, such as, for example pharmaceutical grades of mannitol, lactose, starch, magnesium stearate, talcum, cellulose derivatives, sodium crosscarmellose, glucose, sucrose, magnesium carbonate or sodium saccharin. Such compositions include solutions, suspensions, tablets, capsules, powders and sustained release formulations, such as, but not limited to, implants and microencapsulated delivery systems, and biodegradable, biocompatible polymers, such as collagen, ethylene vinyl acetate, polyanhydrides, polyglycolic acid, polyorthoesters, polylactic acid and others. Methods for preparation of these compositions are known to those skilled in the art. The contemplated compositions may contain 0.001%-100% active ingredient, in one embodiment, 0.1-85% or 75-95% active ingredient.

The active compounds or pharmaceutically acceptable derivatives may be prepared with carriers that protect the compound against rapid elimination from the body, such as time release formulations or coatings.

The compositions may include other active compounds to obtain desired combinations of properties. The compounds provided herein, or pharmaceutically acceptable derivatives thereof as described herein, may also be advantageously administered for therapeutic or prophylactic purposes together with another pharmacological agent known in the general art to be of value in treating liver diseases. It is to be understood that such combination therapy constitutes a further aspect of the compositions and methods of treatment provided herein.

### Compositions for oral administration

Oral pharmaceutical dosage forms are either solid, gel or liquid. The solid dosage forms are tablets, capsules, granules, and bulk powders. Types of oral tablets include compressed, chewable lozenges and tablets which may be enteric-coated, sugar-coated or film-coated. Capsules may be hard or soft gelatin capsules, while granules and powders may be provided in non-effervescent or effervescent form with the combination of other ingredients known to those skilled in the art.

For example, the formulations can be solid dosage forms, such as capsules or tablets. The tablets, pills, capsules, troches and the like can contain any of the following ingredients, or compounds of a similar nature: a binder; a diluent; a disintegrating agent; a lubricant; a glidant; a sweetening agent; and a flavoring agent.

Examples of binders include microcrystalline cellulose, gum tragacanth, glucose solution, acacia mucilage, gelatin solution, sucrose and starch paste. Lubricants include talc, starch, magnesium or calcium stearate, lycopodium and stearic acid. Diluents include, for example, lactose, sucrose, starch, kaolin, salt, mannitol and dicalcium phosphate. Glidants include, but are not limited to, colloidal silicon dioxide. Disintegrating agents include crosscarmellose sodium, sodium starch glycolate, alginic acid, corn starch, potato starch, bentonite, methylcellulose, agar and carboxymethylcellulose. Coloring agents include, for example, any of the approved certified water soluble FD and C dyes, mixtures thereof; and water insoluble FD and C dyes suspended on alumina hydrate. Sweetening agents include sucrose, lactose, mannitol and artificial sweetening agents such as saccharin, and any number of spray dried flavors. Flavoring agents include natural flavors extracted from plants such as fruits and synthetic blends of compounds which produce a pleasant sensation, such as, but not limited to peppermint and methyl salicylate. Wetting agents include propylene glycol monostearate, sorbitan monooleate, diethylene glycol monolaurate and polyoxyethylene laural ether. Emetic-coatings include fatty acids, fats, waxes, shellac, ammoniated shellac and cellulose acetate phthalates. Film coatings include hydroxyethylcellulose, sodium carboxymethylcellulose, polyethylene glycol 4000 and cellulose acetate phthalate.

If oral administration is desired, the compound could be provided in a composition that protects it from the acidic environment of the stomach. For example, the composition can be formulated in an enteric coating that maintains its integrity in the stomach and releases the active compound in the intestine. The composition may also be formulated in combination with an antacid or other such ingredient.

When the dosage unit form is a capsule, it can contain, in addition to material of the above type, a liquid carrier such as a fatty oil. In addition, dosage unit forms can contain various other materials which modify the physical form of the dosage unit, for example, coatings of sugar and other enteric agents. The compounds can also be administered as a component of an elixir, suspension, syrup, wafer, sprinkle, chewing gum or the like. A syrup may contain, in addition to the active compounds, sucrose as a sweetening agent and certain preservatives, dyes and colorings and flavors.

The active materials can also be mixed with other active materials which do not impair the desired action, or with materials that supplement the desired action, such as antacids, H2 blockers, and diuretics. The active ingredient is a compound or pharmaceutically acceptable derivative thereof as described herein. Higher concentrations, up to about 98% by weight of the active ingredient may be included.

Pharmaceutically acceptable carriers included in tablets are binders, lubricants, diluents, disintegrating agents, coloring agents, flavoring agents, and wetting agents. Enteric-coated tablets, because of the enteric-coating, resist the action of stomach acid and dissolve or disintegrate in the neutral or alkaline intestines. Sugar-coated tablets are compressed tablets to which different layers of pharmaceutically acceptable substances are applied. Film-coated tablets are compressed tablets which have been coated with a polymer or other suitable coating. Multiple compressed tablets are compressed tablets made by more than one compression cycle utilizing the pharmaceutically acceptable substances previously mentioned. Coloring agents may also be used in the above dosage forms. Flavoring and sweetening agents are used in compressed tablets, sugar-coated, multiple compressed and chewable tablets. Flavoring and sweetening agents are especially useful in the formation of chewable tablets and lozenges.

Liquid oral dosage forms include aqueous solutions, emulsions, suspensions, solutions and/or suspensions reconstituted from non-effervescent granules and effervescent preparations reconstituted from effervescent granules. Aqueous solutions include, for example, elixirs and syrups. Emulsions are either oil-in-water or water-in-oil.

Elixirs are clear, sweetened, hydroalcoholic preparations. Pharmaceutically acceptable carriers used in elixirs include solvents. Syrups are concentrated aqueous solutions of a sugar, for example, sucrose, and may contain a preservative. An emulsion is a two-phase system in which one liquid is dispersed in the form of small globules throughout another liquid. Pharmaceutically acceptable carriers used in emulsions are non-aqueous liquids, emulsifying agents and preservatives. Suspensions use pharmaceutically acceptable suspending agents and preservatives. Pharmaceutically acceptable substances used in non-effervescent granules, to be reconstituted into a liquid oral dosage form, include diluents, sweeteners and wetting agents. Pharmaceutically acceptable substances used in effervescent granules, to be reconstituted into a liquid oral dosage form, include organic acids and a source of carbon dioxide. Coloring and flavoring agents are used in all of the above dosage forms.

Solvents include glycerin, sorbitol, ethyl alcohol and syrup. Examples of preservatives include glycerin, methyl and propylparaben, benzoic add, sodium benzoate and alcohol. Examples of non-aqueous liquids utilized in emulsions include mineral oil and cottonseed oil. Examples of emulsifying agents include gelatin, acacia, tragacanth, bentonite, and surfactants such as polyoxyethylene sorbitan monooleate. Suspending agents include sodium carboxymethylcellulose, pectin, tragacanth, Veegum and acacia. Diluents include lactose and sucrose. Sweetening agents include sucrose, syrups, glycerin and artificial sweetening agents such as saccharin. Wetting agents include propylene glycol monostearate, sorbitan monooleate, diethylene glycol monolaurate and polyoxyethylene lauryl ether. Organic acids include citric and tartaric acid. Sources of carbon dioxide include sodium bicarbonate and sodium carbonate. Coloring agents include any of the approved certified water soluble FD and C dyes, and mixtures thereof. Flavoring agents include natural flavors extracted from plants such fruits, and synthetic blends of compounds which produce a pleasant taste sensation.

For a solid dosage form, the solution or suspension, in for example propylene carbonate, vegetable oils or triglycerides, can be encapsulated in a gelatin capsule. Such solutions, and the preparation and encapsulation thereof, are disclosed in U.S. Patent Nos 4,328,245; 4,409,239; and 4,410,545. For a liquid dosage form, the solution, e.g., for example, in a polyethylene glycol, may be diluted with a sufficient quantity of a pharmaceutically acceptable liquid carrier, e.g., water, to be easily measured for administration.

Alternatively, liquid or semi-solid oral formulations may be prepared by dissolving or dispersing the active compound or salt in vegetable oils, glycols, triglycerides, propylene glycol esters (e.g., propylene carbonate) and other such carriers, and encapsulating these solutions or suspensions in hard or soft gelatin capsule shells. Other useful formulations include, but are not limited to, those containing a compound provided herein, a dialkylated mono- or poly-alkylene glycol, including, but not limited to, 1,2-dimethoxymethane, diglyme, triglyme, tetraglyme, polyethylene glycol-350-dimethyl ether, polyethylene glycol-550-dimethyl ether, polyethylene glycol-750-dimethyl ether wherein 350, 550 and 750 refer to the approximate average molecular weight of the polyethylene glycol, and one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, thiodipropionic acid and its esters, and dithiocarbamates.

Other formulations include, but are not limited to, aqueous alcoholic solutions including a pharmaceutically acceptable acetal. Alcohols used in these formulations are any pharmaceutically acceptable water-miscible solvents having one or more hydroxyl groups, including, but not limited to, propylene glycol and ethanol. Acetals include, but are not limited to, di(lower alkyl) acetals of lower alkyl aldehydes such as acetaldehyde diethyl acetal.

Tablets and capsules formulations may be coated as known by those of skill in the art in order to modify or sustain dissolution of the active ingredient. Thus, for example, they may be coated with a conventional enterically digestible coating, such as phenylsalicylate, waxes and cellulose acetate phthalate.

### Injectables, solutions and emulsions

Parenteral administration, generally characterized by injection, either subcutaneously, intramuscularly or intravenously is also contemplated herein. Injectables can be prepared in conventional forms, either as liquid solutions or suspensions, solid forms suitable for solution or suspension in liquid prior to injection, or as emulsions. Suitable excipients are, for example, water, saline, dextrose, glycerol or ethanol. In addition, if desired, the pharmaceutical compositions to be administered may also contain minor amounts of non-toxic auxiliary substances such as wetting or emulsifying agents, pH buffering agents, stabilizers, solubility enhancers, and other such agents, such as for example, sodium acetate, sorbitan monolaurate, triethanolamine oleate and cyclodextrins. Implantation of a slow-release or sustained-release system, such that a constant level of dosage is maintained is also contemplated herein. Briefly, a compound described herein is dispersed in a solid inner matrix, e.g., polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcohol and cross-linked partially hydrolyzed polyvinyl acetate, that is surrounded by an outer polymeric membrane, e.g., polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, that is insoluble in body fluids. The compound diffuses through the outer polymeric membrane in a release rate controlling step. The percentage of active compound contained in such parenteral compositions is highly dependent on the specific nature thereof, as well as the activity of the compound and the needs of the patient.

Parenteral administration of the compositions includes intravenous, subcutaneous and intramuscular administrations. Preparations for parenteral administration include sterile solutions ready for injection, sterile dry soluble products, such as lyophilized powders, ready to be combined with a solvent just prior to use, including hypodermic tablets, sterile suspensions ready for injection, sterile dry insoluble products ready to be combined with a vehicle just prior to use and sterile emulsions. The solutions may be either aqueous or nonaqueous.

If administered intravenously, suitable carriers include physiological saline or phosphate buffered saline (PBS), and solutions containing thickening and solubilizing agents, such as glucose, polyethylene glycol, and polypropylene glycol and mixtures thereof.

Pharmaceutically acceptable carriers used in parenteral preparations include aqueous vehicles, nonaqueous vehicles, antimicrobial agents, isotonic agents, buffers, antioxidants, local anesthetics, suspending and dispersing agents, emulsifying agents, sequestering or chelating agents and other pharmaceutically acceptable substances.

Examples of aqueous vehicles include Sodium Chloride Injection, Ringers Injection, Isotonic Dextrose Injection, Sterile Water Injection, Dextrose and Lactated Ringers Injection. Nonaqueous parenteral vehicles include fixed oils of vegetable origin, cottonseed oil, corn oil, sesame oil and peanut oil. Antimicrobial agents in bacteriostatic or fungistatic concentrations must be added to parenteral preparations packaged in multiple-dose containers which include phenols or cresols, mercurials, benzyl alcohol, chlorobutanol, methyl and propyl p-hydroxybenzoic acid esters, thimerosal, benzalkonium chloride and benzethonium chloride. Isotonic agents include sodium chloride and dextrose. Buffers include phosphate and citrate. Antioxidants include sodium bisulfate. Local anesthetics include procaine hydrochloride. Suspending and dispersing agents include sodium carboxymethylcelluose, hydroxypropyl methylcellulose and polyvinylpyrrolidone. Emulsifying agents include Polysorbate 80 (TWEEN® 80). A sequestering or chelating agent of metal ions includes EDTA. Pharmaceutical carriers also include ethyl alcohol, polyethylene glycol and propylene glycol for water miscible vehicles and sodium hydroxide, hydrochloric acid, citric acid or lactic acid for pH adjustment.

The concentration of the pharmaceutically active compound is adjusted so that an injection provides an effective amount to produce the desired pharmacological effect. The exact dose depends on the age, weight and condition of the patient or animal as is known in the art.

The unit-dose parenteral preparations are packaged in an ampule, a vial or a syringe with a needle. All preparations for parenteral administration must be sterile, as is known and practiced in the art.

Illustratively, intravenous or intraarterial infusion of a sterile aqueous solution containing an active compound is an effective mode of administration. Another embodiment is a sterile aqueous or oily solution or suspension containing an active material injected as necessary to produce the desired pharmacological effect.

Injectables are designed for local and systemic administration. In certain embodiments, a therapeutically effective dosage is formulated to contain a concentration of at least about 0.1 % w/w up to about 90% w/w or more, or more than 1% w/w of the active compound to the treated tissue(s). The active ingredient may be administered at once, or may be divided into a number of smaller doses to be administered at intervals of time. It is understood that the precise dosage and duration of treatment is a function of the tissue being treated and may be determined empirically using known testing protocols or by extrapolation from *in vivo* or *in vitro* test data. It is to be noted that concentrations and dosage values may also vary with the age of the individual treated. It is to be further understood that for any particular patient, specific dosage regimens should be adjusted over time according to the individual need and the professional judgment of the person administering or supervising the administration of the formulations, and that the concentration ranges set forth herein are exemplary only and are not intended to limit the scope or practice of the claimed formulations.

The compound may be suspended in micronized or other suitable form or may be derivatized to produce a more soluble active product or to produce a prodrug. The form of the resulting mixture depends upon a number of factors, including the intended mode of administration and the solubility of the compound in the selected carrier or vehicle. The effective concentration is sufficient for ameliorating the symptoms of the condition and may be empirically determined.

### Lyophilized powders

Of interest herein are also lyophilized powders, which can be reconstituted for administration as solutions, emulsions and other mixtures. They may also be reconstituted and formulated as solids or gels.

The sterile, lyophilized powder is prepared by dissolving a compound described herein, or a pharmaceutically acceptable derivative thereof, in a suitable solvent. The solvent may contain an excipient which improves the stability or other pharmacological component of the powder or reconstituted solution, prepared from the powder. Excipients that may be used include, but are not limited to, dextrose, sorbital, fructose, corn syrup, xylitol, glycerin, glucose, sucrose or other suitable agent. The solvent may also contain a buffer, such as citrate, sodium or potassium phosphate or other such buffer known to those of skill in the art at about neutral pH. Subsequent sterile filtration of the solution followed by lyophilization under standard conditions known to those of skill in the art provides the desired formulation. Generally, the resulting solution will be apportioned into vials for lyophilization. Each vial will contain a single dosage (10-1000 mg or 100-500 mg) or multiple dosages of the compound. The lyophilized powder can be stored under appropriate conditions, such as at about 4 °C to room temperature.

Reconstitution of this lyophilized powder with water for injection provides a formulation for use in parenteral administration. For reconstitution, about 1-50 mg, 5-35 mg or about 9-30 mg of lyophilized powder, is added per mL of sterile water or other suitable carrier. The precise amount depends upon the selected compound. Such amount can be empirically determined.

### Topical administration

Topical mixtures are prepared as described for the local and systemic administration. The resulting mixture may be a solution, suspension, emulsions or the like and are formulated as creams, gels, ointments, emulsions, solutions, elixirs, lotions, suspensions, tinctures, pastes, foams, aerosols, irrigations, sprays, suppositories, bandages, dermal patches or any other formulations suitable for topical administration.

The compounds or pharmaceutically acceptable derivatives thereof may be formulated as aerosols for topical application, such as by inhalation (see, e.g., U.S. Patent Nos. 4,044,126, 4,414,209, and 4,364,923, which describe aerosols for delivery of a steroid useful for treatment of inflammatory diseases, particularly asthma). These formulations for administration to the respiratory tract can be in the form of an aerosol or solution for a nebulizer, or as a microfine powder for insufflation, alone or in combination with an inert carrier such as lactose. In such a case, the particles of the formulation will have diameters of less than 50 microns or less than 10 microns.

The compounds may be formulated for local or topical application, such as for topical application to the skin and mucous membranes, such as in the eye, in the form of gels, creams, and lotions and for application to the eye or for intracisternal or intraspinal application. Topical administration is contemplated for transdermal delivery and also for administration to the eyes or mucosa, or for inhalation therapies. Nasal solutions of the active compound alone or in combination with other pharmaceutically acceptable excipients can also be administered.

These solutions, particularly those intended for ophthalmic use, may be formulated as 0.01% - 10% isotonic solutions, pH about 5-7, with appropriate salts.

### Compositions for other routes of administration

Other routes of administration, such as topical application, transdermal patches, and rectal administration are also contemplated herein.

For example, pharmaceutical dosage forms for rectal administration are rectal suppositories, capsules and tablets for systemic effect. Rectal suppositories are used herein mean solid bodies for insertion into the rectum which melt or soften at body temperature releasing one or more pharmacologically or therapeutically active ingredients. Pharmaceutically acceptable substances utilized in rectal suppositories are bases or vehicles and agents to raise the melting point. Examples of bases include cocoa butter (theobroma oil), glycerin-gelatin, carbowax (polyoxyethylene glycol) and appropriate mixtures of mono-, di- and triglycerides of fatty acids. Combinations of the various bases may be used. Agents to raise the melting point of suppositories include spermaceti and wax. Rectal suppositories may be prepared either by the compressed method or by molding. In certain embodiments, the weight of a rectal suppository is about 2 to 3 gm.

Tablets and capsules for rectal administration are manufactured using the same pharmaceutically acceptable substance and by the same methods as for formulations for oral administration.

### Sustained Release Compositions

Active ingredients such as the compounds described herein can be administered by controlled release means or by delivery devices that are well known to those of ordinary skill in the art. Examples include, those described in U.S. Patent Nos.: 3,845,770; 3,916,899; 3,536,809; 3,598,123; 4,008,719; 5,674,533; 5,059,595; 5,591,767; 5,120,548; 5,073,543; 5,639,476; 5,354,556; 5,639,480; 5,733,566; 5,739,108; 5,891,474; 5,922,356; 5,972,891; 5,980,945; 5,993,855; 6,045,830; 6,087,324; 6,113,943; 6,197,350; 6,248,363; 6,264,970; 6,267,981; 6,376,461; 6,419,961; 6,589,548; 6,613,358 and 6,699,500 . Such dosage forms can be used to provide slow or controlled release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled release formulations known to those of ordinary skill in the art, including those described herein, can be readily selected for use with the active ingredients provided herein. Thus, the compositions provided encompass single unit dosage forms suitable for oral administration such as, but not limited to, tablets, capsules, gelcaps, and caplets that are adapted for controlled release.

All controlled release pharmaceutical products have a common goal of improving drug therapy over that achieved by their non controlled counterparts. Ideally, the use of an optimally designed controlled release preparation in medical treatment is characterized by a minimum of drug substance being employed to cure or control the condition in a minimum amount of time. Advantages of controlled release formulations include extended activity of the drug, reduced dosage frequency, and increased patient compliance. In addition, controlled release formulations can be used to affect the time of onset of action or other characteristics, such as blood levels of the drug, and can thus affect the occurrence of side (e.g., adverse) effects.

Most controlled release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled release of an active ingredient can be stimulated by various conditions including, but not limited to, pH, temperature, enzymes, water, or other physiological conditions or compounds.

The drug may be administered using intravenous infusion, an implantable osmotic pump, a transdermal patch, liposomes, or other modes of administration. A pump may be used (*see,* Sefton, CRC Crit. Ref. Biomed. Eng. 14:201 (1987); Buchwald et al., Surgery 88:507 (1980); Saudek et al., N. Engl. J. Med. 321:574 (1989)). Polymeric materials can be used. A controlled release system can be placed in a patient at an appropriate site determined by a practitioner of skill, *i.e.,* thus requiring only a fraction of the systemic dose (*see*, *e.g*., Goodson, Medical Applications of Controlled Release, vol. 2, pp. 115-138 (1984)). Other controlled release systems are discussed in the review by Langer (Science 249:1527-1533 (1990)). The active ingredient can be dispersed in a solid inner matrix, e.g., polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcohol and cross-linked partially hydrolyzed polyvinyl acetate, that is surrounded by an outer polymeric membrane, e.g., polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, that is insoluble in body fluids. The active ingredient then diffuses through the outer polymeric membrane in a release rate controlling step. The percentage of active ingredient in such parenteral compositions is highly dependent on the specific nature thereof, as well as the needs of the patient.

### Targeted Formulations

The compounds described herein, or pharmaceutically acceptable derivatives thereof, may also be formulated to be targeted to a particular tissue, receptor, or other area of the body of the patient to be treated. Many such targeting methods are well known to those of skill in the art. All such targeting methods are contemplated herein for use in the instant compositions. For examples of targeting methods, see, e.g., U.S. Patent Nos. 6,316,652, 6,274,552, 6,271,359, 6,253,872, 6,139,865, 6,131,570, 6,120,751, 6,071,495, 6,060,082, 6,048,736, 6,039,975, 6,004,534, 5,985,307, 5,972,366, 5,900,252, 5,840,674, 5,759,542 and 5,709,874.

Liposomal suspensions, including tissue-targeted liposomes, such as tumor-targeted liposomes, may also be suitable as pharmaceutically acceptable carriers. These may be prepared according to methods known to those skilled in the art. For example, liposome formulations may be prepared as described in U.S. Patent No. 4,522,811. Briefly, liposomes such as multilamellar vesicles (MLV's) may be formed by drying down egg phosphatidyl choline and brain phosphatidyl serine (7:3 molar ratio) on the inside of a flask. A solution of a compound described herein in phosphate buffered saline lacking divalent cations (PBS) is added and the flask shaken until the lipid film is dispersed. The resulting vesicles are washed to remove unencapsulated compound, pelleted by centrifugation, and then resuspended in PBS.

### Dosage and Unit Dosage Forms

In human therapeutics, the doctor will determine the posology which he considers most appropriate according to a preventive or curative treatment and according to the age, weight, stage of the disease and other factors specific to the patient to be treated. Generally, doses are from about 1 to about 1000 mg per day for an adult, or from about 5 to about 250 mg per day or from about 10 to 50 mg per day for an adult. In certain embodiments, doses are from about 5 to about 400 mg per day or 25 to 200 mg per day per adult. Dose rates of from about 50 to about 500 mg per day are also contemplated.

In certain embodiments, the amount of the compound or composition which will be effective in the prevention or treatment of the liver disease or one or more symptoms thereof will vary with the nature and severity of the disease or condition, and the route by which the active ingredient is administered. The frequency and dosage will also vary according to factors specific for each patient depending on the specific therapy (e.g., therapeutic or prophylactic agents) administered, the severity of the disorder, disease, or condition, the route of administration, as well as age, body, weight, response, and the past medical history of the patient. Effective doses may be extrapolated from dose-response curves derived from *in vitro* or animal model test systems.

Exemplary doses of a composition include milligram or microgram amounts of the MMP inhibitor per kilogram of patient or sample weight (e.g., about 10 micrograms per kilogram to about 50 milligrams per kilogram, about 100 micrograms per kilogram to about 25 milligrams per kilogram, or about 100 microgram per kilogram to about 10 milligrams per kilogram). In certain embodiments, the dosage administered to a patient is between 0.20 mg/kg and 2.00 mg/kg, or between 0.30 mg/kg and 1.50 mg/kg of the patient's body weight.

In certain embodiments, the recommended daily dose range of the MMP inhibitor described herein for the conditions described herein lies within the range of from about 0.1 mg to about 1000 mg per day, given as a single once-a-day dose or as divided doses throughout a day. In one embodiment, the daily dose is administered twice daily in equally divided doses. Specifically, a daily dose range should be from about 10 mg to about 200 mg per day, more specifically, between about 10 mg and about 150 mg per day, or even more specifically between about 25 and about 100 mg per day. It may be necessary to use dosages of the active ingredient outside the ranges disclosed herein in some cases, as will be apparent to those of ordinary skill in the art. Furthermore, it is noted that the clinician or treating physician will know how and when to interrupt, adjust, or terminate therapy in conjunction with patient response.

Different therapeutically effective amounts may be applicable for different diseases and conditions, as will be readily known by those of ordinary skill in the art. Similarly, amounts sufficient to prevent, manage, treat or ameliorate such disorders, but insufficient to cause, or sufficient to reduce, adverse effects associated with the compound described herein are also encompassed by the above described dosage amounts and dose frequency schedules. Further, when a patient is administered multiple dosages of a compound described herein, not all of the dosages need be the same. For example, the dosage administered to the patient may be increased to improve the prophylactic or therapeutic effect of the compound or it may be decreased to reduce one or more side effects that a particular patient is experiencing.

In one embodiment, the dosage of the compound described herein administered to prevent, treat, manage, or ameliorate a disorder, or one or more symptoms thereof in a patient is 0.1 mg/kg, 1 mg/kg, 2 mg/kg, 3 mg/kg, 4 mg/kg, 5 mg/kg, 6 mg/kg, 10 mg/kg, or 15 mg/kg or more of a patient's body weight. In another embodiment, the dosage of the compound provided herein administered to prevent, treat, manage, or ameliorate a disorder, or one or more symptoms thereof in a patient is a unit dose of 0.1 mg to 200 mg, 0.1 mg to 100 mg, 0.1 mg to 50 mg, 0.1 mg to 25 mg, 0.1 mg to 20 mg, 0.1 mg to 15 mg, 0.1 mg to 10 mg, 0.1 mg to 7.5 mg, 0.1 mg to 5 mg, 0.1 to 2.5 mg, 0.25 mg to 20 mg, 0.25 to 15 mg, 0.25 to 12 mg, 0.25 to 10 mg, 0.25 mg to 7.5 mg, 0.25 mg to 5 mg, 0.5 mg to 2.5 mg, 1 mg to 20 mg, 1 mg to 15 mg, 1 mg to 12 mg, 1 mg to 10 mg, 1 mg to 7.5 mg, 1 mg to 5 mg, or 1 mg to 2.5 mg.

In certain embodiments, treatment or prevention can be initiated with one or more loading doses of an MMP inhibitor and/or caspase inhibitor described herein followed by one or more maintenance doses. In such embodiments, the loading dose can be, for instance, about 60 to about 400 mg per day, or about 100 to about 200 mg per day for one day to five weeks. The loading dose can be followed by one or more maintenance doses. In another embodiment, each maintenance does can be, independently, about from about 0.1 mg to about 200 mg per day, in one embodiment, between about 5 mg and about 150 mg per day, in another embodiment, between about 10 and about 80 mg per day, in another embodiment, from about 10 mg to about 200 mg per day, in another embodiment, between about 25 mg and about 150 mg per day, or in yet another embodiment, between about 25 and about 80 mg per day. Maintenance doses can be administered daily and can be administered as single doses, or as divided doses.

In certain embodiments, a dose of the MMP inhibitor described herein can be administered to achieve a steady-state concentration of the active ingredient in blood or serum of the patient. The steady-state concentration can be determined by measurement according to techniques available to those of skill or can be based on the physical characteristics of the patient such as height, weight and age. In certain embodiments, a sufficient amount of a compound provided herein is administered to achieve a steady-state concentration in blood or serum of the patient of from about 300 to about 4000 ng/mL, from about 400 to about 1600 ng/mL, or from about 600 to about 1200 ng/mL. Loading doses can be administered to achieve steady-state blood or serum concentrations of about 1200 to about 8000 ng/mL, or about 2000 to about 4000 ng/mL for one to five days. Maintenance doses can be administered to achieve a steady-state concentration in blood or serum of the patient of from about 300 to about 4000 ng/mL, from about 400 to about 1600 ng/mL, or from about 600 to about 1200 ng/mL.

In certain embodiments, administration of the same compound may be repeated and the administrations may be separated by at least 1 day, 2 days, 3 days, 5 days, 10 days, 15 days, 30 days, 45 days, 2 months, 75 days, 3 months, or 6 months. In other embodiments, administration of the same prophylactic or therapeutic agent may be repeated and the administration may be separated by at least at least 1 day, 2 days, 3 days, 5 days, 10 days, 15 days, 30 days, 45 days, 2 months, 75 days, 3 months, or 6 months.

Described herein are unit dosages comprising a compound, or a pharmaceutically acceptable derivative thereof, in a form suitable for administration. Such forms are described in detail above. In certain embodiments, the unit dosage comprises 1 to 1000 mg, 5 to 250 mg or 10 to 50 mg active ingredient. For example, the unit dosages comprise about 1, 5, 10, 25, 50, 100, 125, 250, 500 or 1000 mg active ingredient. Such unit dosages can be prepared according to techniques familiar to those of skill in the art.

### Articles of manufacture

The compounds or pharmaceutically acceptable derivatives can be packaged as articles of manufacture containing packaging material, a compound or pharmaceutically acceptable derivative thereof described herein, which is used for treatment, prevention or amelioration of one or more symptoms associated with liver disease, and a label that indicates that the compound or pharmaceutically acceptable derivative thereof is used for treatment, prevention or amelioration of one or more symptoms of liver diseases.

The articles of manufacture described herein contain packaging materials. Packaging materials for use in packaging pharmaceutical products are well known to those of skill in the art. See, e.g., U.S. Patent Nos. 5,323,907, 5,052,558 and 5,033,252. Examples of pharmaceutical packaging materials include, but are not limited to, blister packs, bottles, tubes, inhalers, pumps, bags, vials, containers, syringes, bottles, and any packaging material suitable for a selected formulation and intended mode of administration and treatment. A wide array of formulations of the compounds and compositions described herein are contemplated.

### Evaluation of the Activity of the Compounds

The biological activity of the compounds can be demonstrated by methods known to one of skill in the art. For example, Neil Kaplowitz has described mouse models for acute liver injury in Mechanisms in Liver Injury and Emerging Therapeutics published by the American Association for the Study of Liver Diseases (2006), which is incorporated herein by reference in it's entirety.

TNF-α is a cytokine that is implicated in inducing liver injury in a variety of acute and chronic liver diseases such as chronic HCV and acute liver failure. An exemplary *in vivo* model to test pharmacological agents against TNF-α induced injury is the TNF-α / D-Gal model of liver injury in mice. In this model, mice are treated with TNF-α / D-Gal and compound is administered to evaluate its ability to protect against liver damage. The compound is administered either before, at the time of or after the treatment with TNF-α / D-Gal, and followed for a period of approximately 6 hours. Allowing this model to persist past 6 hours is a variation used to determine the improved survival afforded by compound treatment.

Multiple outcome measures are used for this evaluation. One of these is the measurement of levels of the liver enzyme ALT in the blood. Elevated ALT levels are routinely observed in the blood of patients suffering from a variety of liver diseases. ALT measurement is a very common and relevant clinical laboratory test for the extent of liver disease in patients. A second measure involves gross and histological evaluation of liver damage. The extent of liver damage can be graded by examining liver samples prepared and evaluated microscopically by trained observers. In certain embodiments, the liver injury can be sufficiently severe as to cause mortality. In certain embodiments, compounds described herein protect against TNF-α / D-Gal induced liver injury as determined by these parameters. In certain embodiments, compounds described herein protect against Fas induced liver injury as determined by these parameters. In certain embodiments, the compounds described herein show reduction in liver injury and hepatic fibrosis in the bile duct ligation model.

Other models of liver injury include the LPS / D-Gal model the α-Fas induced liver injury model and the Con A model of liver injury. These models are also relevant to human disease. All three models are complementary to one another.

In certain embodiments, the compounds described herein show inhibition of HCV replication in HCV replicon assay.

### COMBINATION THERAPY

In certain embodiments, the MMP inhibitors described herein are administered in combination with one or more second agents known to treat a liver disease. The dosages of the second agents are to be used in the combination therapies are known in the art. In certain embodiments, dosages lower than those which have been or are currently being used to prevent or treat liver disease, such as hepatitis B or C, are used in the combination therapies provided herein. The recommended dosages of second agents can obtained from the knowledge of those of skill. For those second agents that are approved for clinical use, recommended dosages are described in, for example, Schiff's Diseases of the Liver 10th edition (2006), Lippincott, Williams and Wilkins, Hardman et al., eds., 1996, Goodman & Gilman's The Pharmacological Basis Of Basis Of Therapeutics 9th Ed, Mc-Graw-Hill, New York; Physician's Desk Reference (PDR) 57th Ed., 2003, Medical Economics Co., Inc., Montvale, NJ, which are incorporated herein by reference in their entireties.

In various embodiments, the therapies (e.g., a compound provided herein and the second agent) are administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours part. In certain embodiments, two or more therapies are administered within the same patient visit.

In certain embodiments, the compound described herein and the second agent are cyclically administered. Cycling therapy involves the administration of a first therapy (e.g., a first prophylactic or therapeutic agents) for a period of time, followed by the administration of a second therapy (e.g., a second prophylactic or therapeutic agents) for a period of time, followed by the administration of a third therapy (e.g., a third prophylactic or therapeutic agents) for a period of time and so forth, and repeating this sequential administration, i.e., the cycle in order to reduce the development of resistance to one of the agents, to avoid or reduce the side effects of one of the agents, and/or to improve the efficacy of the treatment.

In certain embodiments, a compound described herein and a second agent are administered to a patient, for example, a mammal, such as a human, in a sequence and within a time interval such that the compound provided herein can act together with the other agent to provide an increased benefit than if they were administered otherwise. For example, the second active agent can be administered at the same time or sequentially in any order at different points in time; however, if not administered at the same time, they should be administered sufficiently close in time so as to provide the desired therapeutic or prophylactic effect. In one embodiment, the compound provided herein and the second active agent exert their effect at times which overlap. Each second active agent can be administered separately, in any appropriate form and by any suitable route. In other embodiments, the compound provided herein is administered before, concurrently or after administration of the second active agent.

In certain embodiments, the compound described herein and the second active agent are administered in a cycle of less than about 3 weeks, about once every two weeks, about once every 10 days or about once every week. One cycle can comprise the administration of a compound provided herein and the second agent by infusion over about 90 minutes every cycle, about 1 hour every cycle, about 45 minutes every cycle. Each cycle can comprise at least 1 week of rest, at least 2 weeks of rest, at least 3 weeks of rest. The number of cycles administered is from about 1 to about 12 cycles, more typically from about 2 to about 10 cycles, and more typically from about 2 to about 8 cycles.

In certain embodiments, administration of the same agent may be repeated and the administrations may be separated by at least 1 day, 2 days, 3 days, 5 days, 10 days, 15 days, 30 days, 45 days, 2 months, 75 days, 3 months, or 6 months. In other embodiments, the compound provided herein and the second agent are administered at about 2 to 4 days apart, at about 4 to 6 days apart, at about 1 week part, at about 1 to 2 weeks apart, or more than 2 weeks apart.

In other embodiments, courses of treatment are administered concurrently to a patient, i.e., individual doses of the second agent are administered separately yet within a time interval such that the compound provided herein can work together with the second active agent. For example, one component can be administered once per week in combination with the other components that can be administered once every two weeks or once every three weeks. In other words, the dosing regimens are carried out concurrently even if the therapeutics are not administered simultaneously or during the same day.

The second agent can act additively or synergistically with the compound described herein. In one embodiment, the compound described herein is administered concurrently with one or more second agents in the same pharmaceutical composition. In another embodiment, a compound described herein is administered concurrently with one or more second agents in separate pharmaceutical compositions. In still another embodiment, a compound described herein is administered prior to or subsequent to administration of a second agent. Also contemplated are administration of a compound described herein and a second agent by the same or different routes of administration, e.g., oral and parenteral. In certain embodiments, when the compound described herein is administered concurrently with a second agent that potentially produces adverse side effects including, but not limited to, toxicity, the second active agent can advantageously be administered at a dose that falls below the threshold that the adverse side effect is elicited.

In certain embodiments, a compound described herein is administered in combination with one second agent. In further embodiments, a second agent is administered in combination with two second agents. In still further embodiments, a second agent is administered in combination with two or more second agents.

In combination therapy, effective dosages of two or more agents are administered together, whereas in alternation or sequential-step therapy, an effective dosage of each agent is administered serially or sequentially. The dosages given will depend on absorption, inactivation and excretion rates of the drug as well as other factors known to those of skill in the art. It is to be noted that dosage values will also vary with the severity of the condition to be alleviated. It is to be further understood that for any particular patient, specific dosage regimens and schedules should be adjusted over time according to the individual need and the professional judgment of the person administering or supervising the administration of the compositions.

In certain embodiments, the uses provided herein involve administration of the MMP inhibitor described herein in combination with other agent, such as Intron A, Peginterferon alfa-2a (Pegasys R), Peginteferon alfa-2a + ribavirin (Pegasys and Copegus, *see*, *for example*, Hoofnagle et al. in N. enel. J. Med. 355:23), lamivudine, adefovir, entecavir, emtricitabine (FTC), telbivudine (L-dT), valtorcitabine (Val-LdC), elvucitabine (L-Fd4C), clevudine, Racivir, BAM 205, NOV-205 (BAM 205), HepeX-B, Amdoxovir (DAPD), ANA 380 (LB80380), Pradefovir (Remofovir), EHT 899, Pradefovir, Zadaxin (thymosin-alpha), UT 231-B, EP-HBS, HBV Core, MIV 210, SpecifEx-HepB, Pentacept (L-3'-FD4C), Bay 41-4109 INTM-191 or VX-950 (telaprevir).

In certain embodiments, the second agent is selected from the following:

Protease inhibitors: Examples include Medivir HCV Protease Inhibitor (Medivir/Tobotec); ITMN-191 (InterMune), SCH 503034 (Schering) and VX950 (Vertex). Further examples of protease inhibitors include substrate-based NS3 protease inhibitors (Attwood *et al.,* Antiviral peptide derivatives, PCT WO 98/22496, 1998; Attwood et al., Antiviral Chemistry and Chemotherapy 1999, 10, 259-273; Attwood *et al*., Preparation and use of amino acid derivatives as anti-viral agents, German Patent Pub. DE 19914474; Tung *et al.* Inhibitors of serine proteases, particularly hepatitis C virus NS3 protease, PCT WO 98/17679), including alpha ketoamides and hydrazinoureas, and inhibitors that terminate in an electrophile such as a boronic acid or phosphonate (Llinas-Brunet et al, Hepatitis C inhibitor peptide analogues, PCT WO 99/07734); Non-substrate-based NS3 protease inhibitors such as 2,4,6-trihydroxy-3-nitrobenzamide derivatives (Sudo K. et al., Biochemical and Biophysical Research Communications, 1997, 238, 643-647; Sudo K. et al. Antiviral Chemistry and Chemotherapy, 1998, 9, 186), including RD3-4082 and RD3-4078, the former substituted on the amide with a 14 carbon chain and the latter processing a para-phenoxyphenyl group; and Sch 68631, a phenanthrenequinone, an HCV protease inhibitor (Chu M. et al., Tetrahedron Letters 37:7229-7232, 1996).

SCH 351633, isolated from the fungus Penicillium griseofulvum, was identified as a protease inhibitor (Chu M. et al., Bioorganic and Medicinal Chemistry Letters 9:1949-1952). Eglin c, isolated from leech, is a potent inhibitor of several serine proteases such as S. griseus proteases A and B, α-chymotrypsin, chymase and subtilisin. Qasim M.A. et al., Biochemistry 36:1598-1607, 1997.

U.S. patents disclosing protease inhibitors for the treatment of HCV include, for example, U.S. Patent No. 6,004,933 to Spruce et al. which discloses a class of cysteine protease inhibitors for inhibiting HCV endopeptidase 2; U.S. Patent No. 5,990,276 to Zhang et al. which discloses synthetic inhibitors of hepatitis C virus NS3 protease; U.S. Patent No. 5,538,865 to Reyes et a; WO 02/008251 to Corvas International, Inc, and US 7,169,760, US2005/176648, WO 02/08187 and WO 02/008256 to Schering Corporation. HCV inhibitor tripeptides are disclosed in US Patent Nos. 6,534,523, 6,410,531, and 6,420,380 to Boehringer Ingelheim and WO 02/060926 to Bristol Myers Squibb. Diaryl peptides as NS3 serine protease inhibitors of HCV are disclosed in WO 02/48172 and US 6,911,428 to Schering Corporation. Imidazoleidinones as NS3 serine protease inhibitors of HCV are disclosed in WO 02/08198 and US 6,838,475 to Schering Corporation and WO 02/48157 and US 6,727,366 to Bristol Myers Squibb. U.S. patents 7,109,172; 6,909,000; 6,617,390; 6,608,067; 6,265,380 and international publication no. WO 98/17679 to Vertex Pharmaceuticals and WO 02/48116 to Bristol Myers Squibb also disclose HCV protease inhibitors. Further examples of HCV protease inhibitors are disclosed in U.S. patent nos. 7,153,848; 7,138,376; 7,135,462; 7,132,504; 7,112,601; and U.S. publication nos. 2007/0010455; 2006/0276511; 2006/0257980; 2006/0258720; 2006/0252715 to InterMune, Inc.

Thiazolidine derivatives which show relevant inhibition in a reverse-phase HPLC assay with an NS3/4A fusion protein and NS5A/5B substrate (Sudo K. et al., Antiviral Research, 1996, 32, 9-18), especially compound RD-1-6250, possessing a fused cinnamoyl moiety substituted with a long alkyl chain, RD4 6205 and RD4 6193;

Thiazolidines and benzanilides identified in Kakiuchi N. et al. J. EBS Letters 421, 217-220; Takeshita N. et al. Analytical Biochemistry, 1997, 247, 242-246;

A phenanthrenequinone possessing activity against protease in a SDS-PAGE and autoradiography assay isolated from the fermentation culture broth of Streptomyces sp., Sch 68631 (Chu M. et al., Tetrahedron Letters, 1996, 37, 7229-7232), and Sch 351633, isolated from the fungus Penicillium griseofulvum, which demonstrates activity in a scintillation proximity assay (Chu M. et al., Bioorganic and Medicinal Chemistry Letters 9, 1949-1952);

Helicase inhibitors (Diana G.D. *et al*., Compounds, compositions and methods for treatment of hepatitis C, U.S. Pat. No. 5,633,358; Diana G.D. *et al*., Piperidine derivatives, pharmaceutical compositions thereof and their use in the treatment of hepatitis C, PCT WO 97/36554);

Nucleotide polymerase inhibitors and gliotoxin (Ferrari R. et al. Journal of Virology, 1999, 73, 1649-1654), and the natural product cerulenin (Lohmann V. et al., Virology, 1998, 249, 108-118);

Interfering RNA (iRNA) based antivirals, including short interfering RNA (siRNA) based antivirals, such as Sirna-034 and others described in International Patent Publication Nos. WO/03/070750 and WO 2005/012525, and US Patent Publication No. US 2004/0209831.

Antisense phosphorothioate oligodeoxynucleotides (S-ODN) complementary to sequence stretches in the 5' non-coding region (NCR) of the virus (Alt M. et al., Hepatology, 1995, 22, 707-717), or nucleotides 326-348 comprising the 3' end of the NCR and nucleotides 371-388 located in the core coding region of the HCV RNA (Alt M. et al., Archives of Virology, 1997, 142, 589-599; Galderisi U. et al., Journal of Cellular Physiology, 1999, 181, 251-257);

Inhibitors of IRES-dependent translation (Ikeda N *et al*., Agent for the prevention and treatment of hepatitis C, Japanese Patent Pub. JP-08268890; Kai Y. *et al.* Prevention and treatment of viral diseases, Japanese Patent Pub. JP-10101591);

Ribozymes, such as nuclease-resistant ribozymes (Maccjak, D. J. et al., Hepatology 1999, 30, abstract 995) and those disclosed in U.S. Patent No. 6,043,077 to Barber et al.*,* and U.S. Patent Nos. 5,869,253 and 5,610,054 to Draper et al.; and

Nucleoside analogs described in International Publication Nos. WO 01/90121 and WO 01/92282; WO 01/32153; WO 01/60315; WO 02/057425; WO 02/057287;
WO 02/18404; WO 01/79246; WO 02/32920 and WO 02/48165. Certain US patents and patent applications disclosing the use of nucleoside analogs that can be used as second agents to treat hepatitis C virus include: US 7,202,224; 7,125,855; 7,105,499 and 6,777,395 by Merck & Co., Inc.; US 2006/0040890; 2005/0038240; 2004/0121980; 6,846,810; 6,784,166 and 6,660,721 by Roche; US 2005/0009737; US 2005/0009737; 7,094,770 and 6,927,291 by Pharmasset, Ltd.

PCT Publication No. WO 99/43691 to Emory University, entitled "2'-Fluoronucleosides" discloses the use of certain 2'-fluoronucleosides to treat HCV.

Other miscellaneous compounds including 1-amino-alkylcyclohexanes (U.S. Patent No. 6,034,134 to Gold et al.*),* alkyl lipids (U.S. Pat. No. 5,922,757 to Chojkier et al.), vitamin E and other antioxidants (U.S. Pat. No. 5,922,757 to Chojkier et al.*),* squalene, amantadine, bile acids (U.S. Pat. No. 5,846,964 to Ozeki et al.), N-(phosphonoacetyl)-L-aspartic acid, (U.S. Pat. No. 5,830,905 to Diana et al.*),* benzenedicarboxamides (U.S. Pat. No. 5,633,388 to Diana et al.), polyadenylic acid derivatives (U.S. Pat. No. 5,496,546 to Wang et al.*),* 2',3'-dideoxyinosine (U.S. Pat. No. 5,026,687 to Yarchoan et al.), benzimidazoles (U.S. Pat. No. 5,891,874 to Colacino et al.), plant extracts (U.S. Patent No. 5,837,257 to Tsai *et al*., U.S. Patent No. 5,725,859 to Omer et al., and U.S. Patent No. 6,056,961), and piperidenes (U.S. Patent No. 5,830,905 to Diana et al.).

Any other compounds currently in preclinical or clinical development for treatment of hepatitis C virus can be used in combination with the compounds described herein. In certain embodiments, compounds that can be used in combination with the MMP inhibitors described herein include: Interleukin-10 by Schering-Plough, IP-501 by Interneuron, Merimebodib (VX-497) by Vertex, AMANTADINE® (Symmetrel) by Endo Labs Solvay, HEPTAZYME® by RPI, XTL-002 by XTL., HCV/MF59 by Chiron, CIVACIR® (Hepatitis C Immune Globulin) by NABI, LEVOVIRIN® by ICN/Ribapharm, VIRAMIDINE® by ICN/Ribapharm, ZADAXIN® (thymosin alpha-1) by Sci Clone, thymosin plus pegylated interferon by Sci Clone, CEPLENE® (histamine dihydrochloride) by Maxim, VX 950 / LY 570310 by Vertex/Eli Lilly, ISIS 14803 by Isis Pharmaceutical/Elan, JTK 003 by AKROS Pharma, BILN-2061 by Boehringer Ingelheim, CellCept (mycophenolate mofetil) by Roche, T67, a β-tubulin inhibitor, by Tularik, a therapeutic vaccine directed to E2 by Innogenetics, FK788 by Fujisawa Healthcare, Inc., IdB 1016 (Siliphos, oral silybin-phosphatdylcholine phytosome), RNA replication inhibitors (VP50406) by ViroPharma/Wyeth, therapeutic vaccine by Intercell, therapeutic vaccine by Epimmune/Genencor, IRES inhibitor by Anadys, ANA 245 and ANA 246 by Anadys, immunotherapy (Therapore) by Avant, protease inhibitor by Corvas/SChering, helicase inhibitor by Vertex, fusion inhibitor by Trimeris, T cell therapy by CellExSys, polymerase inhibitor by Biocryst, targeted RNA chemistry by PTC Therapeutics, Dication by Immtech, Int., protease inhibitor by Agouron, protease inhibitor by Chiron/Medivir, antisense therapy by AVI BioPharma, antisense therapy by Hybridon, hemopurifier by Aethlon Medical, therapeutic vaccine by Merix, protease inhibitor by Bristol-Myers Squibb/Axys, Chron-VacC, a therapeutic vaccine, by Tripep, UT 231B by United Therapeutics, protease, helicase and polymerase inhibitors by Genelabs Technologies, IRES inhibitors by Immusol, R803 by Rigel Pharmaceuticals, INFERGEN® (interferon alphacon-1) by InterMune, OMNIFERON® (natural interferon) by Viragen, ALBUFERON® by Human Genome Sciences, REBIF® (interferon beta-1a) by Ares-Serono, Omega Interferon by BioMedicine, interferon gamma, interferon tau, and Interferon gamma- 1b by InterMune.

In one embodiment, one or more compounds described herein can be administered in combination or alternation with the therapy of hepatitis C currently available or is being currently developed. In one embodiment, one or more compounds described herein can be administered in combination or alternation with an anti-hepatitis C virus interferon, such as Intron A^{®} (interferon alfa-2b) and Pegasys^{®} (Peginterferon alfa-2a); Roferon A^{®} (Recombinant interferon alfa-2a), Infergen^{®} (consensus interferon;interferon alfacon-1), PEG-Intron^{®} (pegylated interferon alfa-2b) and Pegasys^{®} (pegylated interferon alfa-2a).

In one embodiment, the anti-hepatitis C virus interferon is infergen, IL-29 (PEG-Interferon lambda), R7025 (Maxy-alpha), Belerofon, Oral Interferon alpha, BLX-883 (Locteron), omega interferon, multiferon, medusa interferon, Albuferon or REBIF^{®}.

In one embodiment, one or more compounds described herein can be administered in combination or alternation with an anti-hepatitis C virus polymerase inhibitor, such as ribavirin, viramidine, NM 283 (valopicitabine), R7128 / PSI-6130, R1626, HCV-796 or R1479.

In certain embodiments, the one or more compounds described herein can be administered in combination with ribavarin and an anti-hepatitis C virus interferon, such as Intron A^{®} (interferon alfa-2b) and Pegasys^{®} (Peginterferon alfa-2a); Roferon A^{®} (Recombinant interferon alfa-2a), Infergen^{®} (consensus interferon;interferon alfacon-1), PEG-Intron^{®} (pegylated interferon alfa-2b) and Pegasys^{®} (pegylated interferon alfa-2a).

In certain embodiments, RO-113-0830 is administered in combination with an anti-hepatitis C virus interferon, such as Intron A^{®} (interferon alfa-2b) and Pegasys^{®} (Peginterferon alfa-2a); Roferon A^{®} (Recombinant interferon alfa-2a), Infergen^{®} (consensus interferon;interferon alfacon-1), PEG-Intron^{®} (pegylated interferon alfa-2b) and Pegasys^{®} (pegylated interferon alfa-2a). In certain embodiments, RO-113-0830 is administered in combination with ribavarin. In certain embodiments, RO-113-0830 is administered in combination with ribavarin and an anti-hepatitis C virus interferon, such as Intron A^{®} (interferon alfa-2b) and Pegasys^{®} (Peginterferon alfa-2a); Roferon A^{®} (Recombinant interferon alfa-2a), Infergen^{®} (consensus interferon;interferon alfacon-1), PEG-Intron^{®} (pegylated interferon alfa-2b) and Pegasys^{®} (pegylated interferon alfa-2a).

In one embodiment, one or more compounds described herein can be administered in combination or alternation with an anti-hepatitis C virus protease inhibitor such as ITMN-191, SCH 503034, VX950 (telaprevir) or Medivir HCV Protease Inhibitor.

In one embodiment, one or more compounds described herein can be administered in combination or alternation with an anti-hepatitis C virus vaccine, such as TG4040, PeviPROTM, CGI-5005, HCV/MF59, GV1001, IC41 or INNO0101 (E1).

In one embodiment, one or more compounds described herein can be administered in combination or alternation with an anti-hepatitis C virus monoclonal antibody, such as AB68 or XTL-6865 (formerly HepX-C); or an anti-hepatitis C virus polyclonal antibody, such as cicavir.

In one embodiment, one or more compounds described herein can be administered in combination or alternation with an anti-hepatitis C virus immunomodulator, such as Zadaxin^{®} (thymalfasin), NOV-205 or Oglufanide.

In one embodiment, one or more compounds described herein can be administered in combination or alternation with Nexavar, doxorubicin, PI-88, amantadine, JBK-122, VGX-410C, MX-3253 (Ceglosivir), Suvus (BIVN-401 or virostat), PF-03491390 (formerly IDN-6556), G126270, UT-231B, DEBIO-025, EMZ702, ACH-0137171, MitoQ, ANA975, AVI-4065, Bavituxinab (Tarvacin), Alinia (nitrazoxanide) or PYN 17.

It has been recognized that drug-resistant variants of HBV can emerge after prolonged treatment with an antiviral agent. Drug resistance most typically occurs by mutation of a gene that encodes for an enzyme used in the viral life cycle, and most typically in the case of HBV, DNA polymerase. Recently, it has been demonstrated that the efficacy of a drug against HBV infection can be prolonged, augmented, or restored by administering the compound in combination or alternation with a second, and perhaps third, antiviral compound that induces a different mutation from that caused by the principle drug. Alternatively, the pharmacokinetics, biodistribution, or other parameter of the drug can be altered by such combination or alternation therapy. In general, combination therapy is typically preferred over alternation therapy because it induces multiple simultaneous stresses on the virus.

The anti-hepatitis B viral activity of the compounds described herein, can be enhanced by administering two or more of these compounds in combination or alternation. Alternatively, for example, one or more compounds described herein can be administered in combination or alternation with any other known anti-hepatits B virus agent, such as entecivir, cis-2-hydroxymethyl-5-(5-fluorocytosin-1-yl)-1,3-oxathiolane, preferably substantially in the form of the (-)-optical isomer ("FTC", *see* WO 92/14743); the (-)-enantiomer of cis-2-hydroxymethyl-5-(cytosin-1-yl)-1,3-oxathiolane (3TC); β-D-1,3-dioxolane purine nucleosides as described in U.S. Pat. Nos. 5,444,063 and 5,684,010; β-D-dioxolane nucleosides such as β-D-dioxolanyl-guanine (DXG), β-D-dioxolanyl-2,6-diaminopurine (DAPD), and β-D-dioxolanyl-6-chloropurine (ACP), L-FDDC (5-fluoro-3'-thia-2',3'-dideoxycytidine), L-enantiomers of 3'-fluoro-modified .beta.-2'-deoxyribonucleoside 5'-triphosphates, carbovir, interferon, penciclovir and famciclovir, L-FMAU, famciclovir, penciclovir, BMS-200475, bis pom PMEA (adefovir, dipivoxil); lobucavir, ganciclovir, ribavarin, INTM-191, VX-950 (telaprevir), or any other compound that exhibits an EC₅₀ of less than 15 micromolar in 2.2.15 cells; or their prodrugs or pharmaceutically acceptable salts. Several other examples of anti-HBV agents are provided in U.S. Application Publication No. 20050080034, which incorporated by reference in its entirety.

In another embodiment, a compound described herein is administered in combination or alternation with an immune modulator or other pharmaceutically active modifier of viral replication, including a biological material such as a protein, peptide, oligonucleotide, or gamma globulin, including but not limited to interfereon, interleukin, or an antisense oligonucleotides to genes which express or regulate hepatitis B replication.

Any method of alternation can be used that provides treatment to the patient. Non-limiting examples of alternation patterns include 1-6 weeks of administration of an effective amount of one agent followed by 1-6 weeks of administration of an effective amount of a second agent. The alternation schedule can include periods of no treatment. Combination therapy generally includes the simultaneous administration of an effective ratio of dosages of two or more active agents.

The compounds described herein can also be administered in combination with antibiotics, other antiviral compounds, antifungal agents or other pharmaceutical agents administered for the treatment of secondary infections.

It is understood that the foregoing detailed description and accompanying examples are merely illustrative,

### EXAMPLES

### Preparation of RO-113-0830

2,7-dioxa-spiro[3.5]nonane-1-one (10.8 g), which can be prepared as described in U.S. patent no. 5,932,595, is dissolved in N,N-dimethylformamide (95 mL) and slowly added to a solution containing the sodium salt of 4-(4-chlorophenoxy)thiophenol (generated by the addition of sodium hydride powder (2.14 g, 89.2 mmol) to a solution of 4-(4-chlorophenoxy)thiophenol (15.83 g, 66.8 mmol) in N,N-dimethylformamide (19 mL) at 0 °C and stirring for 30 minutes) over a 10-15 minute period, and then stirred an additional 15 minutes. The resulting slurry is heated to 40 °C, stirred for 5 minutes, tert-butanol (2 mL) is added, and the mixture cooled to room temperature over 20 minutes. The majority of the N,N-dimethylformamide is removed in vacuo, the pH adjusted to 9.2, the resultant slurry diluted with 30% diethyl ether-hexanes (120 mL) and filtered. The filter cake is washed with additional portions of ether (3 times 70 mL), acidified to pH 3.5 with 2N aqueous hydrochloric acid, and extracted into methylene chloride (4 x 350 mL). The combined organic layers are dried over magnesium sulfate, concentrated in vacuo. The solid residue is recrystallized from the minimum amount of methylene chloride-hexanes to afford pure 4-[4-(4-chlorophenoxy)phenylthiomethyl]tetrahydropyran-4-carboxylic acid.

### In vivo evaluation of RO-113-0830

*In vivo* efficacy of RO-113-0830 was assessed using male C57B1/6 mice (Simonsen Labs) in two well-established models of liver damage. The mice were allowed to acclimate for at least three days.

In the TNF-α model of liver injury, D-Galactosamine (D-Gln) (800 mg/kg) and TNFα (20 or 40 µg/kg) were injected IP. RO-113-0830 (0.001-30 mg/kg) was administered PO by gavage 30 minutes before insult. Six hours later, animals were anesthetized with Nembutal (50 mg/kg IP), and blood was taken by cardiac puncture. Plasma ALT activity was determined using a kit from Sigma-Aldrich. RO-113-0830 dose-dependently decreased plasma ALT activity in the TNF-α model. The average ED₅₀ from 4 studies was 0.26 ± 0.08 mg/kg.

To determine a benefit on survival, TNF-α model, D-Galactosamine (D-Gln) (800 mg/kg) and TNF-α (20 or 40 µg/kg) were injected IP, and mice were allowed to survive for 24 hours post insult. All morbid mice were euthanized with 125 mg/kg of Nembutal IP. The average 24 hour survival from 3 studies was 27 ± 7.3% and. 55 ± 7.6% (p=0.03) in the TNFα/D-Gln control mice and RO-113-0830-treated mice, respectively.

In a Fas driven model of liver damage, an activating antibody to Fas (Jo-2) was administered IV. Six hours later, animals were anesthetized with Nembutal (50 mg/kg IP), and blood was taken by cardiac puncture. Plasma ALT activity was determined using a kit from Sigma-Aldrich. RO-113-0830 (10 mg/kg; PO) significantly reduced Fas-induced elevation in plasma ALT activity by an average of 50 % in 2 studies (p<0.05 in each study).

The results of these studies demonstrate that, in certain embodiments, RO-113-0830 is protective in the presence of two important pro-inflammatory cytokines involved in liver diseases. Reduction of liver damage and inflammation was determined by reduction of plasma ALT levels relative to control animals. ALT is a clinically relevant marker of liver damage and is used routinely to assess the extent of on going liver damage and inflammation in patients. In addition, RO-113-0830 demonstrated a survival benefit following administration of TNF-α.

### Inhibition of HCV replication in replicon assay

A Huh7 human hepatoma cell line (the 21-5 cell line), *see,* Pietschmann, T. et al., J. Virol. 76, 2002, 4008-4021, that contains a full-length HCV replicon with three cell culture-adaptive mutations was used in this study to demonstrate the ability of RO-113-0830 to inhibit the replication of HCV RNA replicon in cells. The assay was conducted as described by Pietschmann, T. *et al*., *supra.*

The effects of RO-113-0830 at six half-log concentrations, each in quadruplicate were examined in the HCV RNA replicon antiviral evaluation assay. Human interferon alpha-2b was included in each run as a positive control compound. Subconfluent cultures of the ET line were plated out into 96-well plates that were dedicated for the analysis of cell numbers (cytotoxicity) or antiviral activity and the next day drugs were added to the appropriate wells. Cells were processed 72 hr later when the cells were still sub-confluent. HCV RNA replicon levels and the toxic concentration of drug that reduces cell numbers, as indicated by host cell ribosomal RNA (rRNA) levels, were assessed by TaqMan RT-PCR. The EC₅₀ (concentration inhibition virus replication by 50%), IC₅₀ (concentration decreasing cell viability by 50%) and SI₅₀ (selective index: IC₅₀/EC₅₀) values were calculated.

RO 113-0830 dose dependently inhibited HCV replication achieving 50% inhibition (EC₅₀) at a concentration of 70nM. The IC₅₀ for cytotoxicity in this assay was approximately 25 µm, thus achieving a selectivity index (IC₅₀/E₅₀) of approximately 350. These data demonstrate that, in certain embodiments, RO 113-0830 achieves potent inhibition of hepatitis C virus replication at doses that do not impact the viability of cells.

### In vitro studies in Bile Duct Ligation Model

The bile duct ligation model is a well characterized model of liver fibrosis. Briefly, C57/BL6 wild-type mice 6 to 8 weeks of age were subjected to bile duct ligation (BDL) for 14 days. Sham-operated wild type mice were used as controls. Either RO 113-0830 or CMC (carboxymethylcellulose) were administered by gavage in a dose of 10 mg/kg body weight once a day. Hepatocyte apoptosis was quantified by the TUNEL assay and immunofluorescence for activated caspases 3/7. Liver injury was assessed by histopathology, and quantification of bile infarcts. Hepatic fibrosis was assessed by Sirius red staining and quantitative morphometry. Real-time polymerase chain reaction (PCR) was used to measure mRNA transcripts for collagen 1alpha (I) and alpha-smooth muscle actin.

Following 14 days of BDL, wild type mice treated with RO 113-0830 demonstrated a 3-fold decrease in TUNEL and a 5-fold decrease in caspase 3/7-positive hepatocytes (p <0.01) as compared to animals treated with the vehicle. Histologic examination of livers from BDL wild type animals treated with RO 113-0830 also demonstrated a >70% reduction in the number of bile infarcts as compared to vehicle treated BDL mice. Hepatic transcripts for alpha-smooth muscle actin, a marker for stellate cell activation, and collagen I were increased 6- and 8-fold in 14-day BDL mice as compared to sham-operated controls. The mRNA for these transcripts were reduced by >60% in RO 113-0830 vs. vehicle-treated BDL animals. Sirius red staining of hepatic collagen was also reduced 3-fold in BDL wt mice treated with RO 113-0830. Finally, overall animal survival following 14 days of BDL was also significantly enhanced in the group receiving active drug (p<0.05). These data demonstrate that, in certain embodiments, liver injury and hepatic fibrosis are reduced upon treatment with the MMP inhibitor RO 113-0830.

## Claims

1. A matrix metalloproteinase inhibitor for use in treating chronic viral hepatitis C, wherein the matrix metalloproteinase inhibitor is:

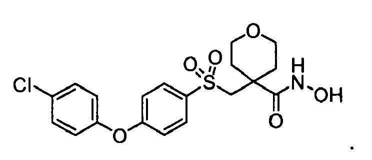

2. The matrix metalloproteinase inhibitor for use according to claim 1, wherein the matrix metalloproteinase inhibitor is prepared to be administered to a patient who has been pre-treated with other medication for the liver disease, or who is being treated with other medication for the liver disease.

3. The matrix metalloproteinase inhibitor for use according to claim 1, wherein the matrix metalloproteinase inhibitor is for use in reducing a liver damage associated with chronic viral hepatitis C.

4. The matrix metalloproteinase inhibitor for use according to claim 1, wherein the matrix metalloproteinase inhibitor is for use in inhibiting hepatitis C virus replication in a cell infected with hepatitis C virus.

5. The matrix metalloproteinase inhibitor for use according to claim 1, wherein the matrix metalloproteinase inhibitor is for use in inhibiting hepatitis C virus replication in a patient infected with hepatitis C virus.

6. The matrix metalloproteinase inhibitor for use according to claim 1 or 2, wherein the matrix metalloproteinase inhibitor is prepared to be administered in addition to a second agent.

7. The matrix metalloproteinase inhibitor for use according to claim 6, wherein the second agent is selected from anti-hepatitis C virus interferon, ribavirin or a combination thereof.

## Patentansprüche

1. Matrix-Metalloproteinaseinhibitor zur Verwendung bei der Behandlung von chronischer viraler Hepatitis C, wobei der Matrix-Metalloproteinaseinhibitor 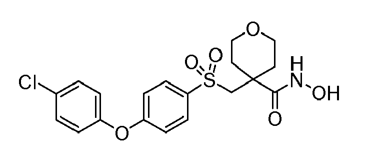 ist.

2. Matrix-Metalloproteinaseinhibitor zur Verwendung nach Anspruch 1, wobei der Matrix-Metalloproteinaseinhibitor zur Verabreichung an einen Patienten, der mit einer anderen Medikation für die Lebererkrankung vorbehandelt worden ist, oder der mit einer anderen Medikation für die Lebererkrankung behandelt wird, hergerichtet ist.

3. Matrix-Metalloproteinaseinhibitor zur Verwendung nach Anspruch 1, wobei der Matrix-Metalloproteinaseinhibitor zur Verwendung bei der Minderung einer mit chronischer viraler Hepatitis C in Zusammenhang stehenden Leberschädigung ist.

4. Matrix-Metalloproteinaseinhibitor zur Verwendung nach Anspruch 1, wobei der Matrix-Metalloproteinaseinhibitor zur Verwendung bei der Hemmung der Replikation des Hepatitis C-Virus in einer mit Hepatitis C-Virus infizierten Zelle ist.

5. Matrix-Metalloproteinaseinhibitor zur Verwendung nach Anspruch 1, wobei der Matrix-Metalloproteinaseinhibitor zur Verwendung bei der Hemmung der Replikation des Hepatitis C-Virus in einem mit Hepatitis C-Virus infizierten Patienten ist.

6. Matrix-Metalloproteinaseinhibitor zur Verwendung nach Anspruch 1 oder 2, wobei der Matrix-Metalloproteinaseinhibitor zur Verabreichung zusätzlich zu einem zweiten Mittel hergerichtet ist.

7. Matrix-Metalloproteinaseinhibitor zur Verwendung nach Anspruch 6, wobei das zweite Mittel aus Anti-Hepatitis C-Virus-Interferon, Ribavirin oder einer Kombination davon ausgewählt ist.

## Revendications

1. Un inhibiteur de métalloprotéinase matricielle pour utilisation dans le traitement de l'hépatite C virale chronique, où l'inhibiteur de métalloprotéinase matricielle est:

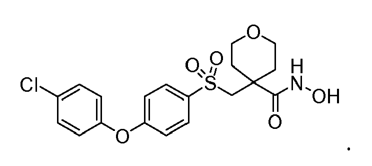

2. L'inhibiteur de métalloprotéinase matricielle pour l'utilisation selon la revendication 1, où l'inhibiteur de métalloprotéinase matricielle est préparé afin d'être administré à un patient qui a été prétraité avec une autre médication pour la maladie hépatique ou qui est traité avec une autre médication pour la maladie hépatique.

3. L'inhibiteur de métalloprotéinase matricielle pour l'utilisation selon la revendication 1, où l'inhibiteur de métalloprotéinase matricielle est pour l'utilisation dans la réduction d'une lésion hépatique associée à l'hépatite C virale chronique.

4. L'inhibiteur de métalloprotéinase matricielle pour l'utilisation selon la revendication 1, où l'inhibiteur de métalloprotéinase matricielle est pour l'utilisation dans l'inhibition de la réplication du virus de l'hépatite C dans une cellule infectée par le virus de l'hépatite C.

5. L'inhibiteur de métalloprotéinase matricielle pour l'utilisation selon la revendication 1, où l'inhibiteur de métalloprotéinase matricielle est pour l'utilisation dans l'inhibition de la réplication du virus de l'hépatite C chez un patient infecté par le virus de l'hépatite C.

6. L'inhibiteur de métalloprotéinase matricielle pour l'utilisation selon la revendication 1 ou 2, où l'inhibiteur de métalloprotéinase matricielle est préparé afin d'être administré en supplément à un deuxième agent.

7. L'inhibiteur de métalloprotéinase matricielle pour l'utilisation selon la revendication 6, où le deuxième agent est sélectionné parmi interférone anti-virus de l'hépatite C, ribavirine ou une combinaison de celles-ci.
